(19) **Europäisches Patentamt / European Patent Office / Office européen des brevets**

(11) **EP 4 322 856 B1**

(12) **EUROPEAN PATENT SPECIFICATION**

(45) Date of publication and mention of the grant of the patent:
**18.02.2026 Bulletin 2026/08**

(21) Application number: **22754981.3**

(22) Date of filing: **13.07.2022**

(51) International Patent Classification (IPC):
**A61B 6/00** $^{(2024.01)}$        **A61B 6/03** $^{(2006.01)}$
**A61B 6/42** $^{(2024.01)}$

(52) Cooperative Patent Classification (CPC):
**A61B 6/032; A61B 6/4233; A61B 6/4241;**
**A61B 6/482; A61B 6/5217; A61B 6/5235**

(86) International application number:
**PCT/US2022/036965**

(87) International publication number:
**WO 2023/287885 (19.01.2023 Gazette 2023/03)**

(54) **SYSTEM FOR GENERATING LOW-ENERGY VIRTUAL MONOENERGETIC IMAGES FROM MULTI-ENERGY COMPUTED TOMOGRAPHY DATA**

SYSTEM ZUR ERZEUGUNG VON ENERGIEARMEN VIRTUELLEN MONOENERGETISCHEN BILDERN AUS MULTIENERGIE-COMPUTERTOMOGRAPHIEDATEN

SYSTÈME POUR GÉNÉRER DES IMAGES MONO-ÉNERGÉTIQUES VIRTUELLES À FAIBLE ÉNERGIE À PARTIR DE DONNÉES DE TOMODENSITOMÉTRIE MULTI-ÉNERGIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.07.2021 US 202163221427 P**

(43) Date of publication of application:
**21.02.2024 Bulletin 2024/08**

(73) Proprietor: **Mayo Foundation for Medical Education and Research**
**Rochester, Minnesota 55905 (US)**

(72) Inventors:
- **GONG, Hao**
  **Rochester, Minnesota 55901 (US)**
- **MCCOLLOUGH, Cynthia H.**
  **Byron, Minnesota 55920 (US)**
- **FLETCHER, Joel G.**
  **Oronoco, Minnesota 55960 (US)**
- **LENG, Shuai**
  **Rochester, Minnesota 55906 (US)**

(74) Representative: **Samson & Partner Patentanwälte mbB**
**Widenmayerstraße 6**
**80538 München (DE)**

(56) References cited:
**WO-A1-2020/006352**

- **GONG HAO ET AL: "Deep-learning-based direct synthesis of low-energy virtual monoenergetic images with multi-energy CT", JOURNAL OF MEDICAL IMAGING, SOCIETY OF PHOTO-OPTICAL INSTRUMENTATION ENGINEERS, 1000 20TH ST. BELLINGHAM WA 98225-6705 USA, vol. 8, no. 5, 19 April 2021 (2021-04-19), pages 52104, XP060147042, ISSN: 2329-4302, [retrieved on 20210419], DOI: 10.1117/1.JMI.8.5.052104**
- **SHENGZHEN TAO ET AL: "Improving iodine contrast to noise ratio using virtual monoenergetic imaging and prior-knowledge-aware iterative denoising (mono-PKAID)", PHYSICS IN MEDICINE AND BIOLOGY, INSTITUTE OF PHYSICS PUBLISHING, BRISTOL GB, vol. 64, no. 10, 16 May 2019 (2019-05-16), pages 105014, XP020336020, ISSN: 0031-9155, [retrieved on 20190516], DOI: 10.1088/1361-6560/AB17FA**

**Description**

CROSS-REFERENCE TO RELATED APPLICATIONS

This application is based on, claims the benefit of, and claims priority to U.S. Provisional Application No. 63/221,427, filed July 13, 2021.

STATEMENT REGARDING FEDERALLY SPONSORED RESEARCH

This invention was made with government support under EB016966 and EB028590 awarded by the National Institutes of Health. The government has certain rights in the invention.

BACKGROUND

Multi-energy CT (MECT) provides X-ray attenuation measurements at multiple different X-ray spectra. MECT images can include two or more energy spectra and can be implemented with various combinations of source(s), detector(s), filter(s), etc. Examples can include multi-source, kV switching, multilayer detectors, beam filters and/or modulators, and photon-counting detectors. Techniques for generating virtual monoenergetic images (VMIs) have been developed that attempt to attempt to mimic CT data that would be acquired with a monoenergetic beam of a particular energy. However, such techniques generally amplify image artifacts and noise, especially at low x-ray energy levels, which obscures the underlying anatomical and pathological features. In addition, current commercial techniques do not generate VMI below 40 keV.

The scientific publication "Deep-learning-based direct synthesis of low-energy virtual monoenergetic images with multi-energy CT", GONG HAO ET AL, JOURNAL OF MEDICAL IMAGING, SOCIETY OF PHOTO-OPTICAL INSTRUMENTATION ENGINEERS, Vol. 8, No. 5, discloses an example of state of the art synthesis of monoergetic X-ray images.

Accordingly, new systems, methods, and media for generating low-energy virtual monoenergetic images from multi-energy computed tomography data are desirable.

SUMMARY OF THE INVENTION

The invention is defined by a system for transforming computed tomography data according to claim 1 with optional features being presented in the dependent claims.

SUMMARY OF THE DISCLOSURE

In accordance with some examples of the disclosed subject matter, systems for generating low-energy virtual monoenergetic images from multi-energy computed tomography data are provided.

In accordance with some examples of the disclosed subject matter, a system for transforming computed tomography data is provided, the system comprising: at least one hardware processor configured to: receive input indicative of a particular X-ray energy level at which to generate a virtual monoenergetic image (VMI) of a subject that includes a plurality of target materials with improved contrast; receive computed tomography (CT) data of a subject; generate a VMI version of the CT data at the particular X-ray energy; and cause the VMI version of the CT data to be presented.

In some examples, the least one hardware processor is further configured to: provide the CT data to a trained model; receive output from the trained model; and generate the VMI version of the CT data based on the output of the trained model.

In some examples, the trained model is a trained convolutional neural network (CNN) that was trained using CT data of one or more subjects, each representing at least one material of a plurality of materials, and synthesized monoenergetic CT (MCT) images of at least one of the one or more subjects phantom based on X-ray attenuation of the plurality of materials.

In some examples, the CT data comprises multi-energy computed tomography (MECT) image data of the one or more subjects.

In some examples, the CT data comprises multi-energy computed tomography (MECT) projection domain data of the one or more subjects.

In some examples, the CT data comprises a VMI at an energy level of at least 40 kilo-electronvolts (keV) of the one or more subjects, and the particular X-ray energy is below 40 keV.

In some examples, the one or more subjects includes a phantom comprising a plurality of samples, each of the plurality of samples representing at least one material of a plurality of materials.

In some examples, the trained model is a least squares model.

In some examples, the particular X-ray energy is above 40 keV.

In some examples, the CT data is MECT image data.

In some examples, the input indicative of the particular X-ray energy level at which to generate the VMI of the subject comprises input explicitly indicating the plurality of target materials.

In some examples, the at least one hardware processor configured to:

identify the particular X-ray energy as an X-ray energy at which contrast between the plurality of target materials is substantially maximized.

In some examples, the input indicative of the particular X-ray energy level at which to generate the VMI of the subject comprises input explicitly indicating a task associated with the CT data.

In some examples, the at least one hardware processor configured to:

receive, via a user input device, the input explicitly indicating the task associated with the CT data.

In some examples, the at least one hardware processor configured to:

receive, from an electronic medical record system, the input explicitly indicating the task associated with the CT data.

In some examples, the plurality of target materials comprises at least two of: gray matter; white matter; an iodine contrast-media; calcium; a mixture of blood and iodine; adipose tissue; hydroxyapatite; lung; liver; muscle; or blood.

In some examples, the VMI version of the CT data is a 40 keV VMI.

In some examples, the at least one hardware processor is further configured to: generate a second VMI version of the CT data at a second particular X-ray energy.

In some examples, the second VMI version of the CT data is a 50 keV VMI.

In some examples, the second VMI version of the CT data is a VMI at an energy below 40 keV.

In some examples, the at least one hardware processor is further configured to: receive the CT data from a CT scanner.

In some examples, the CT scanner is a dual-energy computed tomography scanner that is configured to generate the CT data.

In some examples, the CT scanner is a photon counting detector computed tomography (PCD-CT) scanner that is configured to generate the CT data.

In some examples, the system further comprises the CT scanner.

In some examples, the at least one hardware processor is further configured to: receive the CT data from the CT scanner over a wide area network.

In some examples, the at least one hardware processor is further configured to: receive the CT data from memory.

BRIEF DESCRIPTION OF THE DRAWINGS

Various objects, features, and advantages of the disclosed subject matter can be more fully appreciated with reference to the following detailed description of the disclosed subject matter when considered in connection with the following drawings, in which like reference numerals identify like elements.

FIG. 1 shows an example of a system for virtual monoenergetic imaging from multi-energy computed tomography data in accordance with some embodiments of the disclosed subject matter.

FIG. 2 shows an example of hardware that can be used to implement a multi-energy computed tomography source, a computing device, and a server, shown in FIG. 1 in accordance with some embodiments of the disclosed subject matter.

FIG. 3 shows an example of a flow for training and using mechanisms for virtual monoenergetic imaging from multi-energy computed tomography data in accordance with some embodiments of the disclosed subject matter.

FIG. 4 shows an example of a process for training and using a convolutional neural network that can be used to implement mechanisms for virtual monoenergetic imaging from multi-energy computed tomography data in accordance with some embodiments of the disclosed subject matter.

FIG. 5 shows an example of a topology of a convolutional neural network that can be used to implement mechanisms for virtual monoenergetic imaging from multi-energy computed tomography data in accordance with some embodiments of the disclosed subject matter.

FIG. 6 shows an example of a flow for training and using mechanisms for virtual monoenergetic imaging from multi-energy computed tomography data in accordance with some embodiments of the disclosed subject matter.

FIG. 7 shows an example of a process for training and using a convolutional neural network that can be used to implement mechanisms for virtual monoenergetic imaging from multi-energy computed tomography data in accordance with some embodiments of the disclosed subject matter.

FIG. 8 shows an example of a topology of a convolutional neural network that can be used to implement mechanisms for virtual monoenergetic imaging from multi-energy computed tomography data in accordance with some embodiments of the disclosed subject matter.

FIG. 9 shows examples of virtual images generated using material maps derived from multi-energy computed tomography data of a porcine abdomen using mechanisms for virtual monoenergetic imaging described herein, and using other techniques, which can be used to generate a virtual monoenergetic image at any suitable energy.

FIG. 10 shows examples of iodine concentration predicted from multi-energy computed tomography data of a phantom using mechanisms for virtual monoenergetic imaging described herein, and using other techniques.

FIG. 11 shows examples of virtual monoenergetic images generated from multi-energy computed tomography data of a phantom using mechanisms for virtual monoenergetic imaging described herein, and using other techniques.

FIG. 12 shows examples of virtual monoenergetic images generated from multi-energy computed tomography data of a human subject using mechanisms for virtual monoenergetic imaging described herein, and using other techniques.

FIG. 13 shows examples of virtual monoenergetic images generated from multi-energy computed tomography data of a human subject using mechanisms for virtual monoenergetic imaging described herein, and a clinical mix image generated by a computed tomography scanner.

FIG. 14 shows an example of a process for generating and using a model that can be used to implement mechanisms for virtual monoenergetic imaging from multi-energy computed tomography data in accordance with some embodiments of the disclosed subject matter.

FIG. 15A shows examples of calculated monoenergetic CT numbers for various materials across a range of x-ray energies.

FIG. 15B shows an example of contrast between gray matter and white matter across a range of x-ray energies.

FIG. 16A shows an example of line profiles for the clinical mix image, and virtual monoenergetic images shown in FIG. 13 at a first position.

FIG. 16B shows another example of line profiles for the clinical mix image, and virtual monoenergetic images shown in FIG. 13 at a second position.

FIG. 17 shows examples of predicted CT number for virtual monoenergetic images generated from multi-energy computed tomography data of phantom materials representing gray matter and white matter using mechanisms for virtual monoenergetic imaging described herein at two different radiation dose levels.

FIG. 18 shows examples of predicted CT number for virtual monoenergetic images generated from multi-energy computed tomography data of phantom materials representing mixtures of iodine and blood using mechanisms for virtual monoenergetic imaging described herein at two different radiation dose levels.

FIG. 19 shows examples of mean-absolute-percent-error in CT number of predicted CT number for virtual monoenergetic images generated from multi-energy computed tomography data of various materials.

FIG. 20A shows examples of a virtual monoenergetic image generated from multi-energy computed tomography data of a human subject using mechanisms for virtual monoenergetic imaging described herein, a clinical mix image generated by a computed tomography scanner, and virtual monoenergetic images generated from multi-energy computed tomography data of the human subject using other techniques.

FIG. 20B shows contrast to noise ratio between two regions of interest in the images shown in FIG. 20A.

FIG. 20C an example of line profiles for the clinical mix image, and virtual monoenergetic images shown in FIG. 20A.

FIG. 21A shows additional examples of a virtual monoenergetic image generated from multi-energy computed tomography data of a human subject using mechanisms for virtual monoenergetic imaging described herein, a clinical mix image generated by a computed tomography scanner, and virtual monoenergetic images generated from multi-energy computed tomography data of the human subject using other techniques.

FIG. 21B shows contrast to noise ratio between two regions of interest in the images shown in FIG. 21A.

FIG. 21C an example of line profiles for the clinical mix image, and virtual monoenergetic images shown in FIG. 21A.

## DETAILED DESCRIPTION

In accordance with various embodiments, mechanisms (which can, for example, include systems, methods, and media) for generating low-energy virtual monoenergetic images from multi-energy computed tomography data are provided.

In accordance with some embodiments of the disclosed subject matter, mechanisms described herein can use a convolutional neural network (CNN) to perform material decomposition. For example, the CNN can be trained to estimate the density of a material or materials at pixel locations of multi-energy computed tomography (MECT) data.

In some embodiments, a CNN trained in accordance with mechanisms described herein can be used to generate material decomposition data, which can be used in various applications. For example, material decomposition data output by a CNN trained in accordance with mechanisms described herein can be used to generate a virtual monoenergetic image (VMI), which is intended to mimic CT data that would be acquired with a monoenergetic beam (e.g. in which all photons have one particular X-ray energy rather than the photons being spread across a spectrum of energies, such as within a relatively narrow band centered at the particular X-ray energy). In such an example, material decomposition data output by the CNN can be used to determine contributions to the CT data caused by various materials at a particular X-ray energy. Note that the term VMI is used for convenience, and other terms may be used to refer to an image corresponding to a particular X-ray energy, such as virtual monochromatic image.

In accordance with some embodiments of the disclosed subject matter, mechanisms described herein can use a CNN (or multiple CNNs) to transform MECT data into one or more VMIs. For example, the CNN can be trained to predict a contribution to the MECT from a particular X-ray energy component or components.

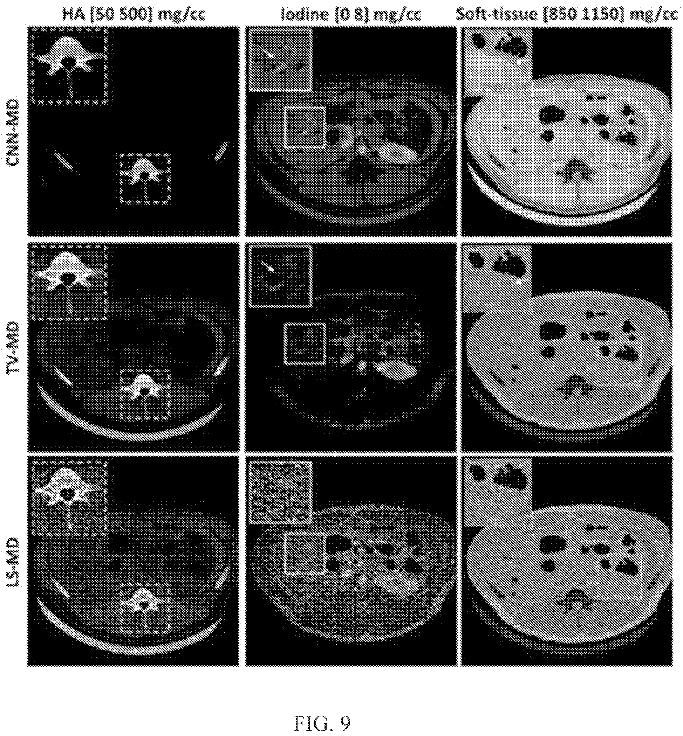

In some embodiments, mechanisms described herein can facilitate reductions in radiation dose, which can lead to reductions in radiation exposure for patients and associated side effects. For example, as described below in connection with FIGS. 9, and 11-13, mechanisms described herein can generate higher quality images (e.g., images with less noise, images with more detail preserved, etc.) than some other techniques at an equivalent dose, which can facilitate reductions in dose without degrading image quality to an unacceptable degree.

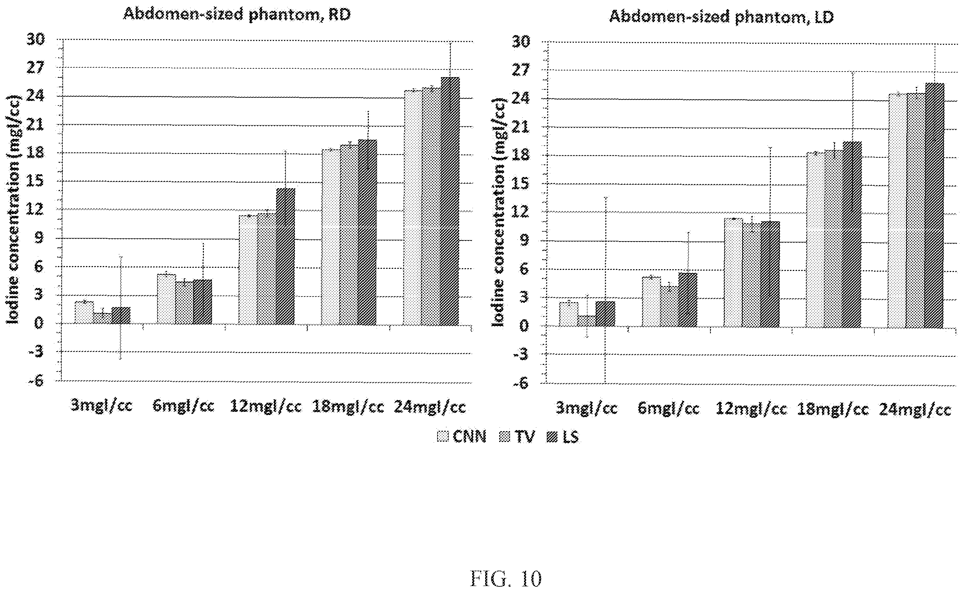

As another example, as described below in connection with FIG. 10, mechanisms described herein can more accurately predict material density than some techniques at an equivalent dose, and can predict material density more accurately than some techniques at a lower dose.

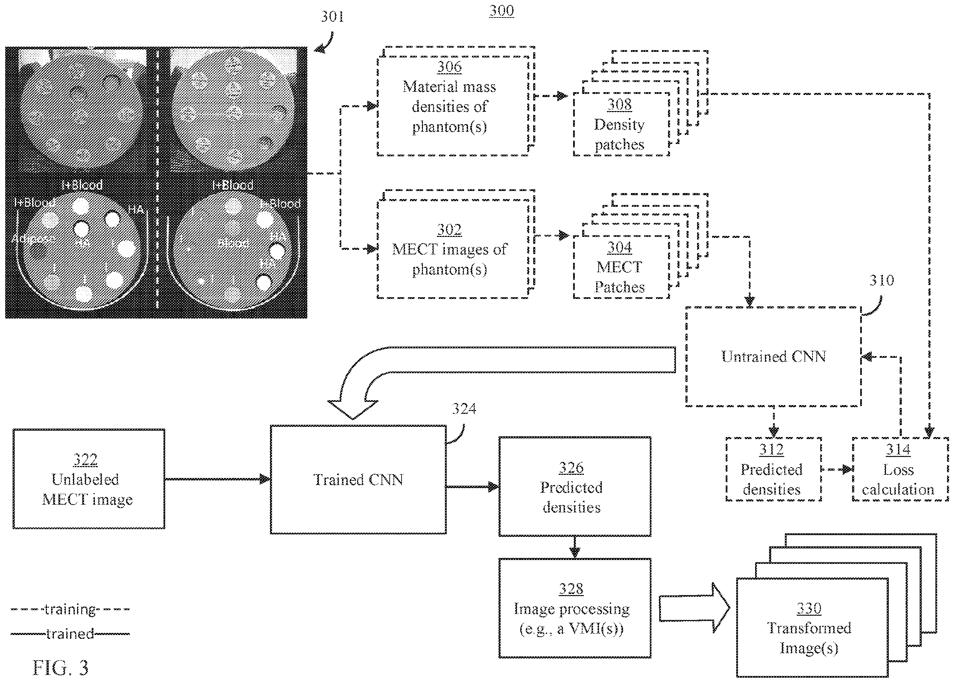

In some embodiments, mechanisms described herein can reduce computational resources utilized to generate VMIs. For example, as described below in connection with FIGS. 3-5, some techniques used to generate VMIs (e.g., using material decomposition techniques) require hours of computational time, while mechanisms described herein can utilize a trained CNN to generate one or more VMIs in less than one minute (e.g., on the order of seconds).

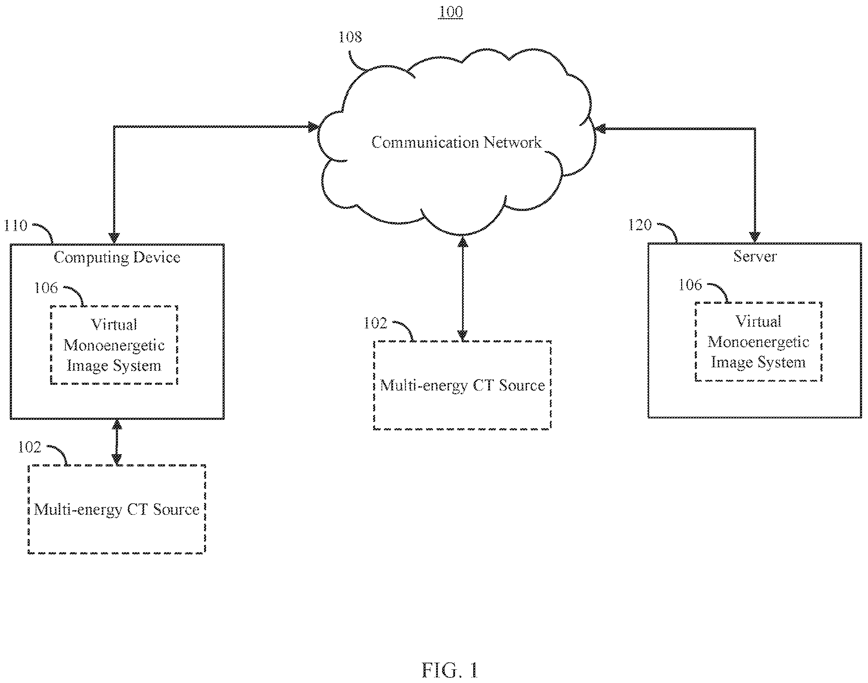

FIG. 1 shows an example 100 of a system for virtual monoenergetic imaging from multi-energy computed tomography in accordance with some embodiments of the disclosed subject matter. As shown in FIG. 1, a computing device 110 can receive multiple MECT data from MECT source 102.

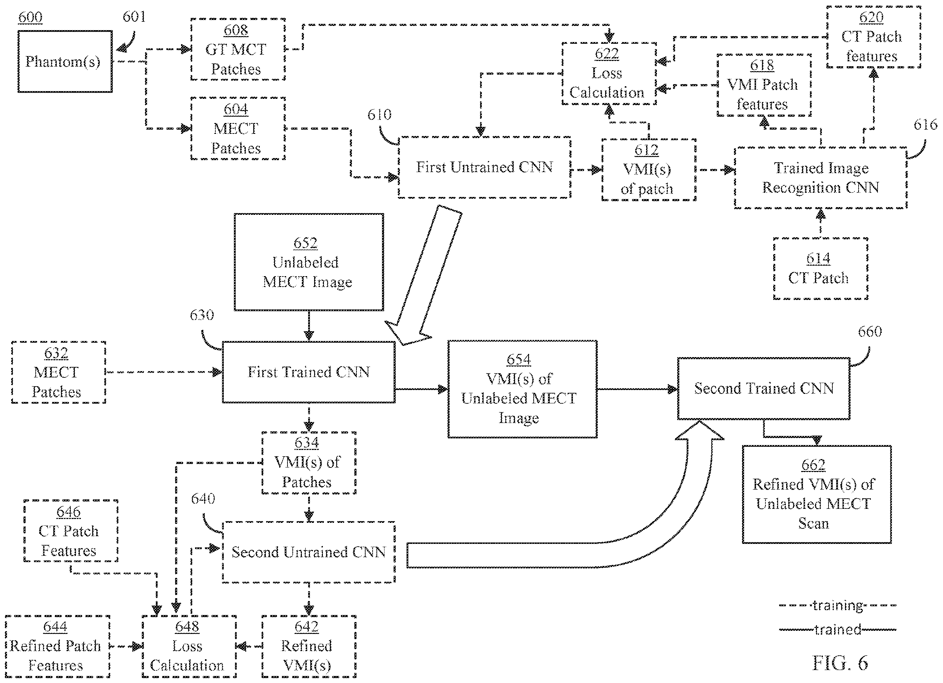

In some embodiments, computing device 110 can execute at least a portion of a virtual monoenergetic image (VMI) system 106 to generate VMIs based on MECT data received from MECT source 102. As described below in connection with FIGS. 3 and 6, VMI system 106 can generate a VMI based on material decomposition data (e.g., received from a material decomposition system, generated by VMI system 106) derived from MECT data, and/or can generate a VMI(s) directly from MECT data.

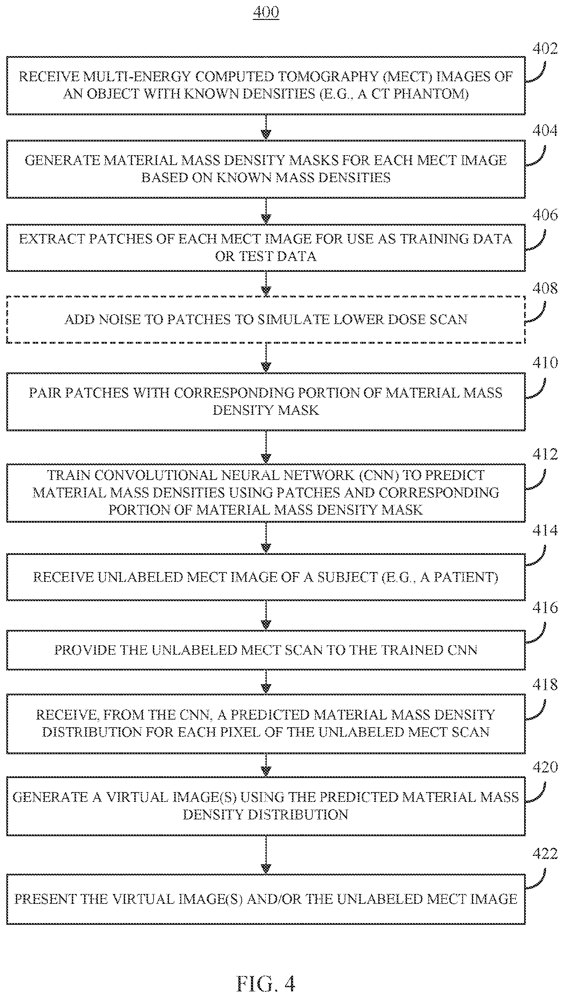

Additionally or alternatively, in some embodiments, computing device 110 can communicate information about MECT data received from MECT source 102 to a server 120 over a communication network 108, which can execute at least a portion of VMI system 106. In such embodiments, server 120 can return information to computing device 110 (and/or any other suitable computing device) indicative of an output VMI system 106 to generate virtual images, to quantify materials, etc. In some embodiments, VMI system 106 can execute one or more portions of process 400 described below in connection with FIG. 4, and/or one or more portions of process 600 described below in connection with FIG. 6.

In some embodiments, computing device 110 and/or server 120 can be any suitable computing device or combination of devices, such as a desktop computer, a laptop computer, a smartphone, a tablet computer, a wearable computer, a server computer, a virtual machine being executed by a physical computing device, etc.

In some embodiments, MECT source 102 can be any suitable source of MECT data. For example, MECT source 102 can be a CT scanner, such as a dual energy CT machine, a photon-counting detector CT (PCD-CT), an energy-integrating-detector (EID)-based MECT machine that generates data at more than two energies, a CT scanner configured with various filters (e.g., spatial spectral filters) to achieve multi-energy CT measurements, etc. As another example, MECT source 102 can be another computing device (e.g., a server storing MECT data). In some embodiments, MECT source 102 can be local to computing device 110. For example, MECT source 102 can be incorporated with computing device 110 (e.g., computing device 110 can be configured as part of a device for capturing and/or storing MECT data). As another example, MECT source 102 can be connected to computing device 110 by a cable, a direct wireless link, etc. Additionally or alternatively, in some embodiments, MECT image source 102 can be located locally and/or remotely from computing device 110, and can communicate MECT data to computing device 110 (and/or server 120) via a communication network (e.g., communication network 108). Note that, in some embodiments, MECT source 102 can provide non-MECT data (e.g., virtual monoenergetic CT data), generated from MECT data within MECT source 102.

In some embodiments, communication network 108 can be any suitable communication network or combination of communication networks. For example, communication network 108 can include a Wi-Fi network (which can include one or more wireless routers, one or more switches, etc.), a peer-to-peer network (e.g., a Bluetooth network), a cellular network (e.g., a 3G network, a 4G network, a 5G network, etc., complying with any suitable standard, such as CDMA, GSM, LTE, LTE Advanced, NR, etc.), a wired network, etc. In some embodiments, communication network 108 can be a local area network, a wide area network, a public network (e.g., the Internet), a private or semi-private network (e.g., a corporate or university intranet), any other suitable type of network, or any suitable combination of networks. Communications links shown in FIG. 1 can each be any suitable communications link or combination of communications links, such as wired links, fiber optic links, Wi-Fi links, Bluetooth links, cellular links, etc.

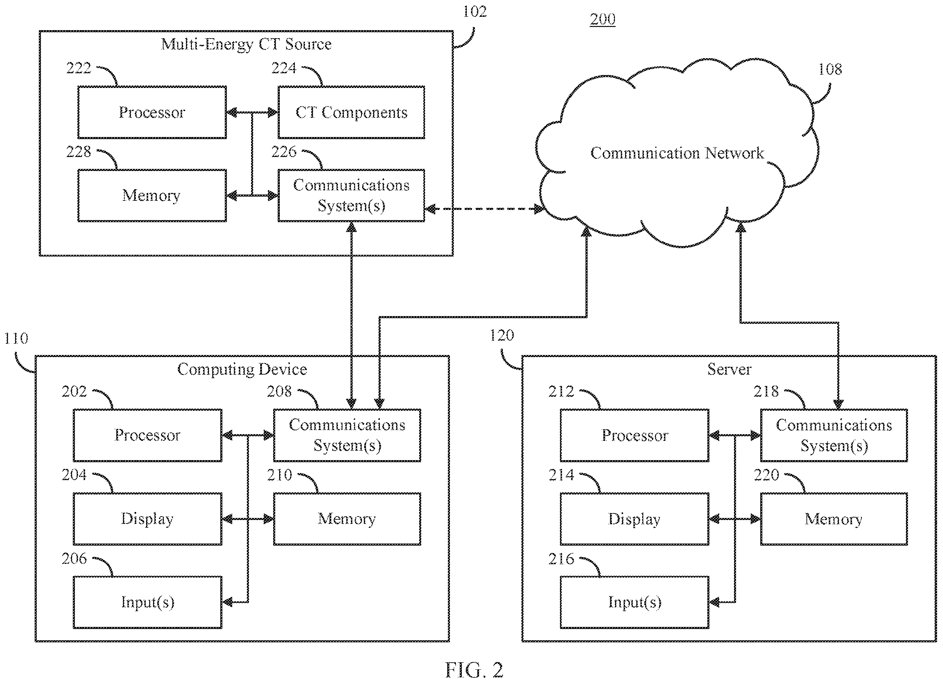

FIG. 2 shows an example 200 of hardware that can be used to implement MECT source 102, computing device 110, and/or server 120 in accordance with some embodiments of the disclosed subject matter. As shown in FIG. 2, in some embodiments, computing device 110 can include a processor 202, a display 204, one or more inputs 206, one or more communication systems 208, and/or memory 210. In some embodiments, processor 202 can be any suitable hardware processor or combination of processors, such as a central processing unit (CPU), a graphics processing unit (GPU), an application specific integrated circuit (ASIC), a field-programmable gate array (FPGA), etc. In some embodiments, display 204 can include any suitable display devices, such as a computer monitor, a touchscreen, a television, etc. In some embodiments, inputs 206 can include any suitable input devices and/or sensors that can be used to receive user input, such as a keyboard, a mouse, a touchscreen, a microphone, etc.

In some embodiments, communications systems 208 can include any suitable hardware, firmware, and/or software for communicating information over communication network 108 and/or any other suitable communication networks. For example, communications systems 208 can include one or more transceivers, one or more communication chips and/or chip sets, etc. In a more particular example, communications systems 208 can include hardware, firmware and/or software that can be used to establish a Wi-Fi connection, a Bluetooth connection, a cellular connection, an Ethernet connection, etc.

In some embodiments, memory 210 can include any suitable storage device or devices that can be used to store instructions, values, etc., that can be used, for example, by processor 202 to present content using display 204, to communicate with server 120 via communications system(s) 208, etc. Memory 210 can include any suitable volatile memory, non-volatile memory, storage, or any suitable combination thereof. For example, memory 210 can include RAM, ROM, EEPROM, one or more flash drives, one or more hard disks, one or more solid state drives, one or more optical drives, etc. In some embodiments, memory 210 can have encoded thereon a computer program for controlling operation of computing device 110. In such embodiments, processor 202 can execute at least a portion of the computer program to generate material decomposition data, generate transformed images (e.g., virtual images, such as VMIs, etc.), present content (e.g., CT images, MECT images, VMIs, material maps, user interfaces, graphics, tables, etc.), receive content from server 120, transmit information to server 120, etc.

In some embodiments, server 120 can include a processor 212, a display 214, one or more inputs 216, one or more communications systems 218, and/or memory 220. In some embodiments, processor 212 can be any suitable hardware processor or combination of processors, such as a CPU, a GPU, an ASIC, an FPGA, etc. In some embodiments, display 214 can include any suitable display devices, such as a computer monitor, a touchscreen, a television, etc. In some embodiments, inputs 216 can include any suitable input devices and/or sensors that can be used to receive user input, such as a keyboard, a mouse, a touchscreen, a microphone, etc.

In some embodiments, communications systems 218 can include any suitable hardware, firmware, and/or software for communicating information over communication network 108 and/or any other suitable communication networks. For example, communications systems 218 can include one or more transceivers, one or more communication chips and/or chip sets, etc. In a more particular example, communications systems 218 can include hardware, firmware and/or software that can be used to establish a Wi-Fi connection, a Bluetooth connection, a cellular connection, an Ethernet connection, etc.

In some embodiments, memory 220 can include any suitable storage device or devices that can be used to store instructions, values, etc., that can be used, for example, by processor 212 to present content using display 214, to communicate with one or more computing devices 110, etc. Memory 220 can include any suitable volatile memory, non-volatile memory, storage, or any suitable combination thereof. For example, memory 220 can include RAM, ROM, EEPROM, one or more flash drives, one or more hard disks, one or more solid state drives, one or more optical drives, etc. In some embodiments, memory 220 can have encoded thereon a server program for controlling operation of server 120. In such embodiments, processor 212 can execute at least a portion of the server program to transmit information and/or content (e.g., MECT data, material decomposition data, VMIs, a user interface, etc.) to one or more computing devices 110, receive information and/or content from one or more computing devices 110, receive instructions from one or more devices (e.g., a personal computer, a laptop computer, a tablet computer, a smartphone, etc.), etc.

In some embodiments, MECT source 102 can include a processor 222, computed tomography (CT) components 224, one or more communications systems 226, and/or memory 228. In some embodiments, processor 222 can be any suitable hardware processor or combination of processors, such as a CPU, a GPU, an ASIC, an FPGA, etc. In some embodiments, CT components 224 can be any suitable components to generate MECT data corresponding to various X-ray energies. An example of an MECT machine that can be used to implement MECT source 102 can include a dual energy CT machine, a PCD-CT machine, etc.

Note that, although not shown, MECT source 102 can include any suitable inputs and/or outputs. For example, MECT source 102 can include input devices and/or sensors that can be used to receive user input, such as a keyboard, a mouse, a touchscreen, a microphone, a trackpad, a trackball, hardware buttons, software buttons, etc. As another example, MECT source 102 can include any suitable display devices, such as a computer monitor, a touchscreen, a television, etc., one or more speakers, etc.

In some embodiments, communications systems 226 can include any suitable hardware, firmware, and/or software for communicating information to computing device 110 (and, in some embodiments, over communication network 108 and/or any other suitable communication networks). For example, communications systems 226 can include one or more transceivers, one or more communication chips and/or chip sets, etc. In a more particular example, communications systems 226 can include hardware, firmware and/or software that can be used to establish a wired connection using any suitable port and/or communication standard (e.g., VGA, DVI video, USB, RS-232, etc.), Wi-Fi connection, a Bluetooth connection, a cellular connection, an Ethernet connection, etc.

In some embodiments, memory 228 can include any suitable storage device or devices that can be used to store instructions, values, MECT data, etc., that can be used, for example, by processor 222 to: control CT components 224, and/or receive CT data from CT components 224; generate MECT data; present content (e.g., MECT images, CT images, VNC images, VNCa images, VMIs, a user interface, etc.) using a display; communicate with one or more computing devices 110; etc. Memory 228 can include any suitable volatile memory, non-volatile memory, storage, or any suitable combination thereof. For example, memory 228 can include RAM, ROM, EEPROM, one or more flash drives, one or more hard disks, one or more solid state drives, one or more optical drives, etc. In some embodiments, memory 228 can have encoded thereon a program for controlling operation of MECT source 102. In such embodiments, processor 222 can execute at least a portion of the program to generate MECT data, transmit information and/or content (e.g., MECT data) to one or more computing devices 110, receive information and/or content from one or more computing devices 110, transmit information and/or content (e.g., MECT data) to one or more servers 120, receive information and/or content from one or more servers 120, receive instructions from one or more devices (e.g., a personal computer, a laptop computer, a tablet computer, a smartphone, etc.), etc.

FIG. 3 shows an example 300 of a flow for training and using mechanisms for virtual monoenergetic imaging from multi-energy computed tomography data in accordance with some embodiments of the disclosed subject matter. In some embodiments, mechanisms for virtual monoenergetic imaging from MECT data can be implemented in the post-reconstruction domain (e.g., after raw CT data has been transformed into image data). For example, such mechanisms can be used to directly convert MECT data (e.g., MECT images) to material mass density distribution data. In a more particular example, such MECT data can be formatted as multiple image sets, with each image set corresponding to the same anatomy at different energies. As another more particular example, such MECT data can be formatted as a single set of images in which each pixel is associated with multiple CT numbers (e.g., one CT number per energy level) of the MECT data. Additionally or alternatively, mechanisms for virtual monoenergetic imaging from MECT data can be implemented in the projection domain (e.g., using raw CT data).

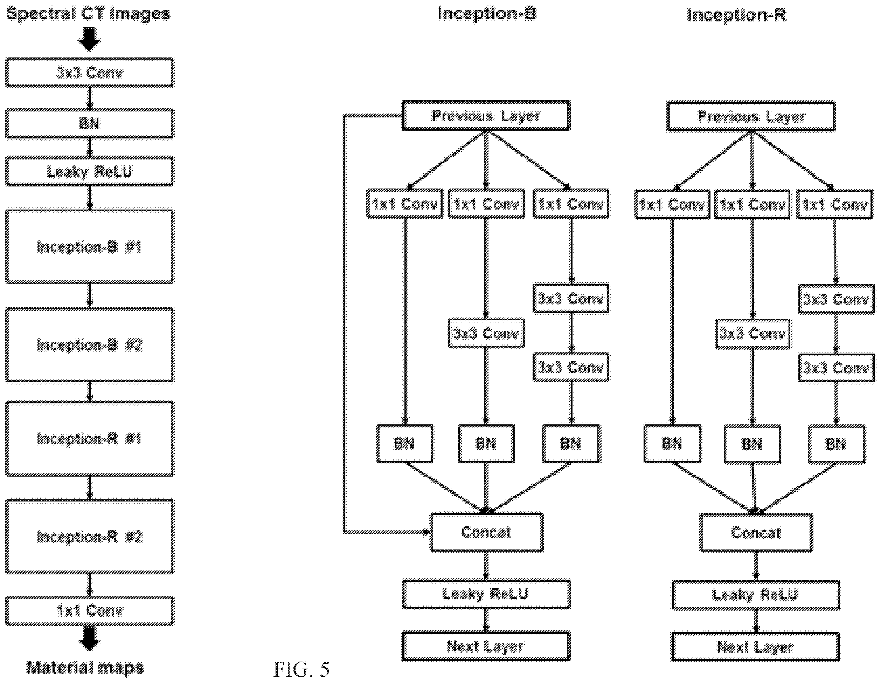

In some embodiments, mechanisms described herein can be used to train a convolutional neural network (CNN) to predict mass densities distributions of basis materials using MECT data as input. As described below in connection with FIG. 5, the CNN can be implemented using functional modules (e.g., functional modules referred to herein as "Inception-B" and "Inception-R") that can improve robustness against local image noise and artifacts (e.g., by exploiting multi-scale local image features). In some embodiments, the CNN can be implemented using a topology that does not include pooling layers, which are often included in CNNs trained to perform tasks related to image processing. Omitting pooling operations can facilitate preservation of anatomical details in the MECT data. Additionally, in some embodiments, mechanisms described herein can utilize a loss function L during training that includes a fidelity term and an image-gradient-correlation (IGC) regularization term. For example, the loss function can be represented using the relationship:

$$L = \frac{1}{N}\sum_{i,j,k}(\|f_{cnn} - f_{GT}\|_2^2 + \frac{1}{\rho(\nabla f_{cnn}, \nabla f_{GT}) + \epsilon}),$$

(1)

where $\nabla f_{i,j,k}$, can be represented using the relationship:

$$\nabla f_{i,j,k} = \left|f_{i+1,j,k} - f_{i,j,k}\right| + \left|f_{i,j+1,k} - f_{i,j,k}\right|.$$

(2)

Note that in EQ. (1), indexes are omitted. For example, in EQ. (1), $f_{cnn}$ is used to represent $f_{cnn_{i,j,k}}$,

In EQ. (1), a fidelity term (i.e., $\|f_{cnn} - f_{GT}\|_2^2$ in EQ. (1)) is the mean square error between a density output $f_{cnn}$ of the CNN for a material $k$ and a pixel at position $i,j$ and a "ground truth" $f_{GT}$ mass density for the same material and pixel, and N is the number of materials in the output. An IGC term (i.e., $\dfrac{1}{\rho(\nabla f_{cnn}, \nabla f_{GT}) + \epsilon}$ in EQ. (1)) is the reciprocal of the correlation between the corresponding image gradients, where $\rho$ can represent a correlation operator (e.g., Pearson's correction), and $\epsilon$ can be a small positive constant that can prevent the denominator from having a zero value. In some embodiments, the IGC term can improve the delineation of boundaries of varying anatomical structure in the mass density distributions.

As shown in FIG. 3, MECT images 302 of one or more human-sized CT phantoms 301 can be generated (e.g., using MECT source 102). In some embodiments, MECT images 302 can be generated at varying radiation dose levels (e.g., from low to high). For example, MECT images 302 can be generated at three dose levels (e.g., a low dose, a regular dose, and a high dose). In a more particular example, a low dose can correspond to a volume CT dose index $(CTDI_{vol})$ of 7 milligrays (mGy), a regular dose can correspond to a $CTDI_{vol}$ of 13 mGy, and a high dose can correspond to a $CTDI_{vol}$ of 23 mGy. However, these are merely examples, and any suitable radiation dose levels and/or number of radiation dose levels can be used to generate MECT images 302. For example, MECT images can be generated at two dose levels, or more than three dose levels. In a particular example, each phantom can be scanned multiple times at each dose level (e.g., 5 times at each of 3 dose levels, for a total of 15 scans per phantom). In some embodiments, MECT images 302 can be generated using a two-energy-threshold data acquisition technique, in which a low threshold $(T_L)$ image and a high threshold $(T_H)$ image are generated, with the $T_L$ image including photons detected above a relatively low energy threshold, and the $T_H$ image including only photons detected above a relatively high energy threshold. In such embodiments, the $T_L$ image can be based on contributions from both high and low energy photons, while the $T_H$ image can be based on contributions from only high energy photons. Note that this is an example, MECT images of a subject (e.g., an animal subject) with known material properties can be used in addition to, or in lieu of, MECT images of phantom 301.

In some embodiments, each CT phantom 301 used to generate training data can include inserts of varying base materials (and/or materials that simulate such base materials), such as blood, bone (for example, hydroxyapatite (HA) is included in one of the phantoms shown in FIG. 3), calcium, fat (e.g., adipose tissue), iodine contrast-media (I in FIG. 3), iodine-blood mixture (I+Blood in FIG. 3), water and air. Additionally, portions of CT phantoms 301 not corresponding to a sample insert can represent soft tissue (e.g., muscle tissue in the human body). In some embodiments, multiple examples of the same base material can be included at different concentrations and/or in different amounts. For example, iodine contrast-media, iodine-blood mixture, and HA are included multiple times. In a more particular example, in the phantom on the left I is provided multiple times with different concentrations, while in the phantom on the right I is provided in different amounts. In some embodiments, in addition to, or in lieu of, images of CT phantom 301, additional training data can be generated by creating synthesized MECT images based on theoretical and/or empirical values of attenuation at various X-ray energies caused by various basis materials. In such an example, a distribution of basis materials mass densities used to generate synthetic training data can be used as labels when training the CNN.

In some embodiments, image patches 304 can be extracted (e.g., by computing device 110, by server 120, by VMI system 106) from MECT images of the phantom(s) (e.g., MECT images 302). For example, image patches 304 can be 64x64 pixels images sampled from MECT images 302 (which can be, e.g., 512x512 pixels). As another example, image patches 304 can represent a relatively small fraction of MECT images (e.g., 1/64, 1/96, 1/128, etc.).

In some embodiments, noise (e.g., a random noise parameter which can added or subtracted from the original value(s)) can be added (e.g., by computing device 110, by server 120, by VMI system 106) to one or more pixels of MECT patches 304. For example, noise can be added to MECT patches extracted from high radiation dose MECT images, which can simulate an MECT image captured at a lower dose. Note that the original MECT patch and/or the MECT patch with added noise can be included in MECT patches 304. In some embodiments, use of real and synthesized low-dose CT data (e.g., MECT patches with noise added) to train the CNN can cause the trained CNN to be more robust to image noise in unlabeled low-dose MECT data (e.g., MECT image data for which densities distributions are unknown and/or not provided to the CNN during training).

In some embodiments, material mass densities 306 can be generated, which can represent the density of one or more materials at a particular location on a phantom 301. For example, material mass densities 306 can include information indicative of a density of the material(s) of the phantom. In some embodiments, material mass densities 306 can be derived by directly measuring the values using the phantom, or can be calculated with a simple calibration step. In some embodiments, a training dataset can be formed (e.g., by computing device 110, by server 120, by VMI system 106) from MECT patches 304 paired with a corresponding theoretical material mass density patch(es) 308 that include a subset of data from material mass densities 306. In some embodiments, a portion of MECT patches 304 can be used as training data (e.g., a training set, which can be divided into a training set and a validation set), and another portion of MECT patches 304 can be used as test data (e.g., a test set), which can be used to evaluate the performance of a CNN after training is halted. For example, 3/5 of MECT patches 304 can be used as training data, and the other 2/5 can be reserved as test data. In some embodiments, MECT patches 304 can be selected for inclusion in the training data as a group (e.g., MECT patches 304 generated from the same MECT image 302 can be included or excluded as a group).

In some embodiments, an untrained CNN 310 can be trained (e.g., by computing device 110, by server 120, by VMI system 106) using MECT patches 304 and density patches 308. In some embodiments, untrained CNN 310 can have any suitable topology, such as a topology described below in connection with FIG. 5. In some embodiments, using patches (e.g., MECT patches 304) to train untrained CNN 310 (e.g., rather than whole images) can increase the number of training samples available for training from a particular set of images, which can improve robustness of the network. Additionally, using patches (e.g., MECT patches 304) can reduce the amount of computing resources used during training. For example, using MECT patches 304 rather than MECT images 302 can reduce the amount of memory used during training.

In some embodiments, untrained CNN 310 can be trained using an Adam optimizer (e.g., based on an optimizer described in Kingma et al., "Adam: A Method for Stochastic Optimization," available at arxiv(dot)org, 2014). As shown in FIG. 3, a particular sample MECT patch 304 can be provided as input to untrained CNN 310, which can output predicted material mass densities 312 for each pixel in MECT patch 304. In some embodiments, MECT patches 304 can be formatted in any suitable format. For example, MECT patches 304 can be formatted as a single image patch in which each pixel is associated with multiple CT numbers, with each CT number corresponding to a particular X-ray energy or range of X-ray energies (e.g., associated with a particular X-ray source, associated with a particular filter, associated with a particular bin, etc.). As another example, a particular MECT patch 304 can be formatted as a set (e.g., of two or more patches) that each represent a particular X-ray energy or range of X-ray energies. In some embodiments, CT numbers associated with different X-ray energies can be input to untrained CNN 310 using different channels. For example, untrained CNN 310 can have a first channel corresponding to a first X-ray energy level (e.g., a particular X-ray energy or a range of X-ray energies), and a second channel corresponding to a second X-ray energy level (e.g., a particular X-ray energy or a range of X-ray energies). In some embodiments, predicted mass densities 312 can formatted in any suitable format. For example, predicted mass densities 312 can be formatted as a set of predicted concentration values for various base materials at each pixel (e.g., each pixel can be associated with predicted concentration values for various base materials and/or combinations of materials). In a more particular example, predicted mass densities 312 can include a predicted concentration for iodine at each pixel (e.g., in milligrams per cubic centimeter (mg/cc)), a predicted concentration for bone at each pixel (e.g., in mg/cc), and a predicted concentration for soft tissue at each pixel (e.g., in mg/cc). In such an example, untrained CNN 310 can have three output channels, with one channel corresponding to a combination of soft tissues (e.g., blood, fat, and muscle). As another more particular example, predicted mass densities 312 can include a predicted concentration for additional base materials at each pixel (e.g., a predicted concentration for iodine at each pixel, a predicted concentration for bone at each pixel, a predicted concentration for blood at each pixel, etc.). Note that the predicted concentration for a particular base material or combination of materials can be zero or a non-zero value.

In some embodiments, predicted densities 312 can be compared to the corresponding density patch(es) 308 to evaluate the performance of untrained CNN 310. For example, a loss value can be calculated using loss function L described above in connection with EQ. (1), which can be used to evaluate the performance of untrained CNN 310 in predicting material mass densities of the particular MECT patch 304. In some embodiments, the loss value can be used to adjust weights of untrained CNN 310. For example, a loss calculation 314 can be performed (e.g., by computing device 110, by server 120, by VMI system 106) to generate a loss value that can represent a performance of untrained CNN 310. The loss value generated by loss calculation 314 can be used to adjust weights of untrained CNN 310.

In some embodiments, after training has converged (and the trained CNN performs adequately on the test data), untrained CNN 310 with final weights can be used to implement as a trained CNN 324. In some embodiments, untrained CNN 310 can be used with larger images (e.g., standard-sized MECT images of 512x512 pixels per image) after training. For example, the architecture of untrained CNN 310 can be configured such that the output is the same size as the input regardless of input size without reconfiguration. In a more particular example, each layer of untrained CNN 310 can output an array that is the same size as the input to that layer.

As shown in FIG. 3, an unlabeled MECT image 322 can be provided as input to trained CNN 324, which can output predicted material mass densities 326 for each pixel in MECT.

In some embodiments, predicted densities 326 can be used (e.g., by computing device 110, by server 120, by VMI system 106) to perform image processing 328 to estimate properties of MECT image 322 and/or generate different versions 330 of MECT image 322. For example, predicted densities 326 can be used to generate one or more virtual non-contrast (VNC) images, which can correspond to a version of MECT image 322 with contributions from a contrast-media (e.g., an iodine contrast-media) removed. As another example, predicted densities 326 can be used to generate one or more virtual non-calcium (VNCa) images, which can correspond to a version of MECT image 322 with contributions from calcium (e.g., cancellous bone) removed. As yet another example, predicted densities 326 can be used to generate one or more virtual monoenergetic images (VMIs), which can represent a version of MECT image 322 that includes only contributions from X-rays within a narrow band of energies (e.g., X-rays of about 40 keV, X-rays of about 50 keV). In some embodiments, transformed images 330 can include one or more VNC images, one or more VNCa images, one or more VMIs, etc. For example, transformed images 330 can represent different versions of MECT image 322 that have been transformed to suppress aspects of the MECT image and/or highlight aspects of the MECT image. Such a transformed image can cause certain features of a subject's anatomy to be presented more clearly than in a conventional CT or MECT image.

In some embodiments, an MECT image (e.g., MECT image 322) can be represented as a set of components each corresponding to a contribution from X-rays in a particular energy band. For example, an MECT image $(CT(E))$ can be represented as a matrix, such that $CT(E) = [CT(E_1) ... CT(E_M)]^\mathsf{T}$, where $CT(E_m)$ represents the contribution of X-rays with energy $E_m$ to the value of each pixel, CT $(E_1)$ represents the contribution of X-rays with an energy $E_1$ (e.g., a lowest level energy) to the value of each pixel, and $CT(E_M)$ represents the contribution of X-rays with an energy $E_m$ (e.g., a highest level energy) to the value of each pixel. For example, $CT(E_m)$ can be an $i \times j$ array of values, each corresponding to a pixel location with CT(E) being i $\times$ j pixels, where $CT(E) = \Sigma_{m \in M} CT(E_m)$. In a more particular example, $CT(E_m)$ can be a 512x512 array representing the contribution to each pixel from X-rays having energy $E_m$.

In some embodiments, an MECT measurement (e.g., an MECT image) can be formulated using the relationship:

$$CT(E) = \begin{bmatrix} CT(E_1) \\ \vdots \\ CT(E_M) \end{bmatrix} = \begin{bmatrix} CT(E_1)_1/\rho_1 & \cdots & CT(E_1)_N/\rho_N \\ \vdots & \vdots & \vdots \\ CT(E_M)_1/\rho_1 & \cdots & CT(E_M)_N/\rho_N \end{bmatrix} \begin{bmatrix} \hat{\rho}_1 \\ \vdots \\ \hat{\rho}_N \end{bmatrix},$$

(3)

where $CT(E_m)_n$ is the CT number (e.g., image pixel value) of material $n$ in a pure form at X-ray energy level $E_m$, $\rho_n$ is a nominal mass density of material $n$ in a pure form, and $\hat{\rho}_n$ is the predicted mass density in a mixed form as predicted by the trained CNN (e.g., $\hat{\rho}_n$ can be a portion of predicted densities 326 output by trained CNN 324). In some embodiments, each component of EQ. (3) can be an array of values, with each element corresponding to a pixel location in original CT image $CT(E)$.

In some embodiments, a transformed image can be generated by aggregating contributions from each material at each energy. For example, a VMI at a given X-ray energy level $E_m$ (e.g., $CT(E_m)_{VMI}$) can be generated by adding all basis materials attenuation (indexed across 1, ..., n, ... N) at $E_m$ where $n$ corresponds to a particular basis material (or combination of materials) and $N$ is the number of predicted density values output by the CNN, using the following relationship:

$$CT(E_m)_{VMI} = \sum_{n \in N} \frac{CT(E_m)_n}{\rho_n} \times \hat{\rho}_n,$$

(5)

Note that a VMI generated using EQ. (5) may present a different noise texture than conventional CT images that are used in clinical practice, as trained CNN 324 can suppress noise from the original MECT image.

FIG. 4 shows an example 400 of a process for training and using a convolutional neural network that can be used to implement mechanisms for virtual monoenergetic imaging from multi-energy computed tomography data in accordance with some embodiments of the disclosed subject matter. As shown in FIG. 4, process 400 receives MECT images of an object with known materials having known mass densities. For example, process 400 can receive MECT images of a phantom with known material properties. In a more particular example, process 400 can receive MECT images 302 of one or more phantoms 301.

At 404, process 400 can generate material mass density masks for each MECT image received at 402 based on the known material properties of the object(s) included in the MECT images. In some embodiments, a material mass density mask can be formatted as a matrix of material mass density values corresponding to each pixel of a particular MECT image (or set of images) received at 402. For example, each material mass density mask generated at 404 can correspond to a particular material, and can be formatted as an $i$ x j matrix, where the MECT image has a size of $i \times$ j pixels. As a more particular example, each material mass density mask generated at 404 can correspond to a particular material, and can be formatted as a 512 $\times$ 512 matrix, where the MECT image has a size of 512 $\times$ 512 pixels. As another example, each material mass density mask generated at 404 can represent multiple materials (e.g., all materials of interest), and can be formatted as an $i \times j \times$ k matrix, where the MECT image has a size of $i \times$ j pixels and there are $k$ materials of interest.

At 406, process 400 can extract patches of each MECT image received at 402 for use as training data and/or test data. In some embodiments, process 400 can extract patches of any suitable size using any suitable technique or combination of techniques. For example, process 400 can extract MECT patches 304 from MECT images 302. As a more particular example, process 400 can extract 64x64 pixel patches from MECT images received at 402. As another more particular example, process 400 can extract patches that include sample materials inserted in the phantom (e.g., samples included in phantoms 301). In such an example, process 400 can extract multiple patches that include at least a portion of the same sample(s). In such an example, these patches may or may not overlap (e.g., may or may depict the same portions of the same sample(s)). Stride length between patches can be variable in each direction. For example, patches that do not include any samples (e.g., patches that include only phantom material, without any insert material, patches that include only air, etc.) can be omitted, and/or the number of such patches used in training can be limited (e.g., to no more than a particular number of examples).

At 408, process 400 can generate additional training and/or test data by adding noise to at least a portion of the patches extracted at 406. In some embodiments, patches for which noise is to be added can be copied before adding noise such that there is an original version of the patch and a noisy version of the patch in the data. In some embodiments, 408 can be omitted.

At 410, process 400 can pair patches extracted at 406 and/or generated at 408 with a corresponding portion(s) of the material density mask(s) generated at 404. For example, process 400 can determine which MECT image the patch was derived from, and which portion of MECT image the patch represents. In such an example, process 400 can generate a material mass density mask(s) that have the same dimensions (e.g., same height and width) as the patch.

At 412, process 400 can train a CNN to predict material mass densities in MECT images using the patches extracted at 406 and/or generated at 408 as inputs, and using the known material mass densities paired with each patch at 410 to evaluate performance of the CNN during training (and/or during testing). In some embodiments, process 400 can use any suitable technique or combination of techniques to train the CNN, such as techniques described above in connection with FIG. 3.

At 414, process 400 can receive an unlabeled MECT image of a subject (e.g., a patient). In some embodiments, the unlabeled MECT image can be received from any suitable source, such as a CT machine (e.g., MECT source 102), a computing device (e.g., computing device 110), a server (e.g., server 120), and/or from any other suitable source.

At 416, process 400 can provide the unlabeled MECT image to the trained CNN.

At 418, process 400 can receive, from the trained CNN, an output that includes predicted material mass density distributions. For example, process 400 can receive an output formatted as described above in connection with predicted densities 326. In another example, process 400 can receive an output formatted as an i $\times$ j $\times$ k matrix, where the MECT image has a size of $i \times j$ pixels and there are $k$ materials of interest, and each element of the matrix represents a predicted density at a particular pixel for a particular material.

At 420, process 400 can generate one or more transformed images (and/or any other suitable information, such as iodine quantification) using any suitable technique or combination of techniques. For example, process 400 can generate one or more transformed images using techniques described above in connection with FIG. 3 (e.g., in connection with transformed images 330).

At 422, process 400 can cause the transformed image(s) generated at 420 and/or the original MECT image received at 414 to be presented. For example, process 400 can cause the transformed image(s) and/or original MECT image to be presented by a computing device (e.g., computing device 110). Additionally or alternatively, in some embodiments, process 400 can cause the transformed image(s) generated at 420, the output received at 418, the original MECT image received at 414, and/or any other suitable information, to be recorded in memory (e.g., memory 210 of computing device 110, memory 220 of server 120, and/or memory 228 of MECT source).

FIG. 5 shows an example of a topology of a convolutional neural network that can be used to implement mechanisms for virtual monoenergetic imaging from multi-energy computed tomography data in accordance with some embodiments of the disclosed subject matter. As shown in FIG. 5, the CNN can include a convolution layer (Conv) (e.g., a 3 $\times$ 3 convolution layer), a batch normalization layer (BN), a leaky rectified linear unit (Leaky ReLU), four functional blocks (e.g., two consecutive Inception-B blocks, and two consecutive Inception-R blocks), and another convolution layer (e.g., a 1 $\times$ 1 convolution layer). The Inception-B blocks can receive an output from a previous layer, which can be provided to three convolution layers in parallel (e.g., three 1 $\times$ 1 convolution layers). One of the convolution layers can provide an output directly to a batch normalization layer, another can be followed by a second convolution layer (e.g., a 3 $\times$ 3 convolution layer) which outputs to a batch normalization layer, and the third can be followed by two convolution layers (e.g., two 3 $\times$ 3 convolution layers) the second of which outputs to a third batch normalization layer. Each batch normalization layer outputs to a concatenation layer, which also directly receives the output from the previous layer via a residual connection. A leaky ReLU can follow the concatenation layer, and can output to a next layer. In general, a leaky ReLU can permit negative values (e.g., when the input is negative).

As shown in FIG. 5, the Inception-R blocks are similar to the Inception-B blocks, but the residual connection from the previous layer to the concatenation layer is omitted.

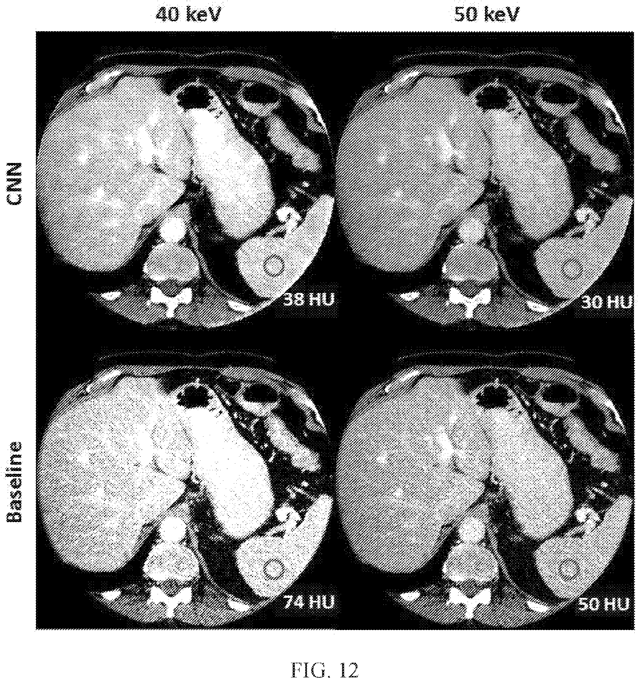

FIG. 6 shows an example 600 of a flow for training and using mechanisms for virtual monoenergetic imaging from multi-energy computed tomography data in accordance with some embodiments of the disclosed subject matter. In some embodiments, mechanisms for virtual monoenergetic imaging from MECT data can be implemented in the post-reconstruction domain to directly convert MECT images to VMI. As described below in connection with FIGS. 12-14, mechanisms described herein for virtual monoenergetic imaging from MECT data can facilitate, for example, reductions in radiation dose, increased image contrast, detail preservation, image artifact suppression.

In some embodiments, mechanisms described herein can be used to train a CNN to predict CT values for one or more VMIs at particular X-ray energies. Additionally, in some embodiments, mechanisms described herein can be used to train a second CNN to output a VMI with improved image quality that is based on the predicted values output by the first CNN. For example, the second CNN can be trained to sharpen features in the VMI output by the first CNN, to improve texture of the VMI output by the first CNN. In some embodiments, the first trained CNN can provide a direct quantitative conversion between MECT images and VMI, and the second trained CNN can improve detail preservation and noise texture similarity between the VMI and the conventional CT images that are widely used in clinical practice. For example, the second trained CNN can adjust values of the predicted VMI output by the first trained CNN to more closely resemble images that practitioners (e.g., radiologists) are accustomed to analyzing. In a particular example, two CNN models can be trained to jointly predict a VMI at preselected X-ray energy levels (e.g., at 40 keV and 50 keV), using MECT images as an initial input.

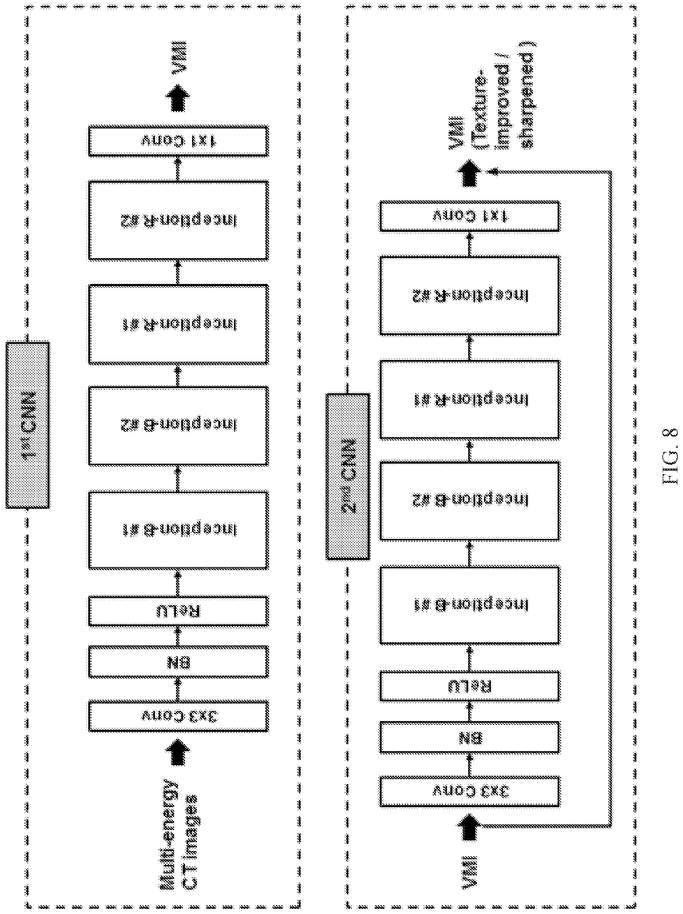

As described below in connection with FIG. 8, the first trained CNN and the second trained CNN can have a similar topology to the CNN described above in connection with FIGS. 3-5, but can be trained using different techniques (e.g., using different labels, using a different loss function, etc.). In some embodiments, the second trained CNN can be omitted, and an output of the first trained CNN can be presented as a VMI corresponding to a particular X-ray energy.

In some embodiments, mechanisms described herein can utilize a second loss function $L(f_{CNN1})$ during training of the first CNN that includes a fidelity term and an image-gradient-correlation (IGC) regularization term, which can be similar to the fidelity term and the IGC regularization term in EQ. (1). Additionally, loss function $L(f_{CNN1})$ can include a feature-reconstruction term. For example, the loss function can be represented using the relationship:

$$L(f_{CNN1}) = \frac{1}{M}\sum_{i,j,m}\left(\left\|f_{CNN,m} - f_{GT,m}\right\|_2^2 + \frac{1}{\frac{\rho_x(\nabla f_{CNN},\nabla f_{GT})}{2} + \frac{\rho_y(\nabla f_{CNN},\nabla f_{GT})}{2} + \epsilon} + \right.$$

$$\left.\lambda_1\left\|\phi(\tilde{f}_{CNN}) - \phi(\tilde{f}_{prior})\right\|_2^2\right),$$

$$(6)$$

where $\nabla f_{i,j,m}$, can be represented using a modified form of EQ. (2). As described above in connection with EQ. (1), indexes are omitted in EQ. (6). For example, in EQ. (6), $f_{cnn,m}$ is used to represent $f_{cnni,j,m}$.

In EQ. (6), a fidelity term (i.e., $\left\|f_{CNN,m} - f_{GT,m}\right\|_2^2$ in EQ. (6)) is the mean square error between a VMI output $f_{CNN,m}$ of the CNN for an X-ray energy m and a pixel at position $i, j$ and a "ground truth" monoenergetic image $f_{GT,m}$ for the same X-ray energy and pixel position. An IGC term (i.e., $\frac{1}{\frac{\rho_x(\nabla f_{cnn},\nabla f_{GT})}{2} + \frac{\rho_y(\nabla f_{cnn},\nabla f_{GT})}{2} + \epsilon}$ in EQ. (6)) is the reciprocal of the correlation between the corresponding image gradients, where $\rho_x$ and $\rho_y$ can represent correlation operators (e.g., Pearson's correction) along different orthogonal dimensions of the images, and E can be a small positive constant that can prevent the denominator from having a zero value. Note that, in some embodiments, the IGC term can be extended to 3D volumetric image data. In some embodiments, the IGC term can improve the delineation of boundaries of varying anatomical structure in the mass density distributions. Note that the fidelity term and IGC term in EQ. (6) are similar to corresponding terms in EQ. (1). A feature-extraction term (i.e., $\lambda_1\left\|\phi(\tilde{f}_{CNN}) - \phi(\tilde{f}_{prior})\right\|_2^2$ in EQ. (6)) is the mean square error between feature maps $\Phi(\tilde{f}_{CNN})$ and $\Phi(\tilde{f}_{prior})$ output by a layer of a pre-trained CNN (e.g., a CNN trained as a general image recognition CNN), where $\Phi$ denotes that the features are output from the pre-trained CNN, and $\lambda_1$ is a relaxation parameter used to adjust the weight of the feature reconstruction loss. In EQ. (6), $f_{CNN}$ can represent an output (e.g., predicted VMI patch 612) generated by the first CNN, and $f_{prior}$ can represent a routine-dose mixed-energy CT image corresponding to the input image (e.g., corresponding to MECT patch 604). Images $\tilde{f}_{CNN}$ and $\tilde{f}_{prior}$ can be generated by applying instance-wise normalization to $f_{CNN}$ and $f_{prior}$, respectively (e.g., instance-wise normalization can implement normalization per training sample). Feature maps $\Phi(\tilde{f}_{CNN})$ and $\Phi(\tilde{f}_{prior})$ can be features output by a hidden layer of the pre-trained CNN when $f_{CNN}$ and $\tilde{f}_{prior}$ are provided as input to the pre-trained CNN, respectively. In some embodiments, the pre-trained general image recognition CNN can be any suitable general image recognition CNN. For example, the pre-trained CNN can be a CNN that was trained using examples from the ImageNet dataset to recognize a variety of objects and/or classify images. In a more particular example, pre-trained CNN can be an instance of a CNN based on the VGG-19 CNN described in Simonyan et al., "Very Deep Convolutional Networks for Large-Scale Image Recognition," available at arxiv(dot)org, 2014.

The second CNN can be trained using a loss function (e.g., $L(f_{CNN2})$) that is similar to the loss function used to train the first CNN (e.g., loss function $L(f_{CNN1})$ described above in connection with FIG. 6), but having a fidelity term that is modified to be the mean square error between the outputs of the first CNN model and the second CNN model (e.g.,

$$\left\| f_{CNN2,m} - f_{CNN,m} \right\|_2^2$$
, where $f_{CNN2,m}$ is the VMI output by the second CNN for an X-ray energy m and a pixel at position *i,j)*. The IGC term can be modified to use the $T_L$ image from MECT patch 632 as the target image (e.g., used to calculate $\nabla f_{GT}$).

In some embodiments, the first CNN and/or the second CNN described in connection with FIGS. 6-8 can be trained using similar techniques to techniques described above in connection with FIGS. 3-5 for training a CNN for VMI. For example, the first CNN can be trained using MECT patches 604, which can be similar (or identical to) MECT patches 304 described above in connection with FIG. 3. In some embodiments, MECT patches 604 can be extracted from MECT images generated using a two-energy-threshold data acquisition technique, in which a low threshold $(T_L)$ image and a high threshold $(T_H)$ image are generated, with the $T_L$ image including photons detected above a relatively low energy threshold, and the $T_H$ image including only photons detected above a relatively high energy threshold. In such embodiments, the $T_L$ image can be based on contributions from both high and low energy photons, while the $T_H$ image can be based on contributions from only high energy photons. In some embodiments, MECT patches 604 can include at least two values corresponding to each pixel. For example, MECT patches 604 can include a Bin 1 value and a Bin 2 value at each pixel. In such an example, the Bin 1 values can include contributions from photons with energies between the low threshold and the high threshold. In a particular example, Bin 1 values can be derived based on a difference between the $T_L$ image and the $T_H$ image (e.g., *Bin 1 = $T_L$ - $T_H$)*. Bin 2 values can include contributions from photons with energies between the high threshold and the tube potential of an X-ray source. In a particular example, Bin 2 values can be identical to the values of the $T_H$ image.

In some embodiments, noise (e.g., a random noise parameter which can be added or subtracted from the original value(s)) can be added (e.g., by computing device 110, by server 120, by VMI system 106) to one or more pixels of MECT patches 604. For example, noise can be added to MECT patches extracted from high radiation dose MECT images, which can simulate an MECT image captured at a lower dose. Note that the original MECT patch and/or the MECT patch with added noise can be included in MECT patches 604. In some embodiments, use of real and synthesized low-dose CT data (e.g., MECT patches with noise added) to train the first CNN can cause the first trained CNN to be more robust to image noise in unlabeled low-dose MECT data (e.g., MECT image data for which densities distributions are unknown and/or not provided to the first CNN during training).

In some embodiments, virtual monoenergetic computed tomography (MCT) images (which are sometimes referred to herein as synthetic MCT images, not shown in FIG. 6) can be generated for each phantom, which can represent a calculated CT value at each pixel if the phantom were images using a particular X-ray energy $E_m$ (e.g., in kiloelectronvolt (keV)). For example, the synthetic MCT images can include information indicative of an average attenuation at each pixel if the phantom were exposed exclusively to X-rays at the particular energy. In some embodiments, synthetic MCT images can be used as a "ground truth" label that can be compared to a VMI generated by the CNN during training. Note that this is an example, MECT images of a subject (e.g., an animal subject) with known material properties can be used in addition to, or in lieu of, MECT images of a phantom.

In some embodiments, a training dataset can be formed (e.g., by computing device 110, by server 120, by VMI system 106) from MECT patches 604 paired with a corresponding synthetic MCT image patch(es) 608 (which are sometimes referred to herein as ground truth MCT patches 608) at one or more X-ray energies that each includes a subset of a synthetic MCT image corresponding to the MECT patch. In some embodiments, a portion of MECT patches 604 can be used as training data (e.g., a training set, which can be divided into a training set and a validation set), and another portion of MECT patches 604 can be used as test data (e.g., a test set), which can be used to evaluate the performance of a CNN after training is halted. For example, 3/5 of MECT patches 604 can be used as training data, and the other 2/5 can be reserved as test data. In some embodiments, MECT patches 604 can be selected for inclusion in the training data as a group (e.g., MECT patches 604 generated from the same MECT image can be included or excluded as a group).

In some embodiments, a first untrained CNN 610 can be trained (e.g., by computing device 110, by server 120, by VMI system 106) using MECT patches 604 and ground truth MCT patches 608. In some embodiments, first untrained CNN 610 can have any suitable topology, such as a topology described below in connection with FIG. 8.

In some embodiments, first untrained CNN 610 can be trained using an Adam optimizer (e.g., based on an optimizer described in Kingma et al., "Adam: A Method for Stochastic Optimization," 2014). As shown in FIG. 6, a particular sample MECT patch 604 can be provided as input to first untrained CNN 610, which can output a predicted VMI patch (or patches) 612. In some embodiments, MECT patches 604 can be formatted in any suitable format. For example, MECT patches 604 can be formatted as a single image patch in which each pixel is associated with multiple CT numbers, with each CT number corresponding to a particular X-ray energy or range of X-ray energies (e.g., associated with a particular X-ray source, associated with a particular filter, associated with a particular bin, etc.). As another example, a particular MECT patch 604 can be formatted as a set (e.g., of two or more patches) that each represent a particular X-ray energy or range of X-ray energies. In some embodiments, CT numbers associated with different X-ray energies can be input to first untrained CNN 610 using different channels. For example, first untrained CNN 610 can have a first channel corresponding to a first X-ray energy level (e.g., a particular X-ray energy or a range of X-ray energies), and a second channel corresponding to a second X-ray energy level (e.g., a particular X-ray energy or a range of X-ray energies).

In some embodiments, MCT patches 608 can be formatted in any suitable format. For example, MCT patches 608 can be formatted as a single image patch in which each pixel is associated with multiple CT numbers, with each CT number corresponding to a particular X-ray energy (e.g., a particular monoenergetic X-ray energy, such as 40 keV, 50 keV, 130 keV etc.). As another example, a particular MCT patch 608 can be formatted as a set (e.g., of two or more patches) that each represent a particular X-ray energy (e.g., a particular monoenergetic X-ray energy, such as 40 keV, 50 keV, 130 keV etc.).

In some embodiments, predicted VMI patch (or patches) 612 output by first untrained CNN 610 can formatted in any suitable format. For example, predicted VMI patch 612 can be formatted as a set of predicted CT values for each of various X-ray energies (e.g., each pixel can be associated with predicted CT values for various X-ray energies, such as 40 keV, 50 keV, etc.). In a more particular example, predicted VMI patch 612 can include a predicted CT number for a first X-ray energy at each pixel, a predicted CT number for a second X-ray energy at each pixel, a predicted CT number for a third X-ray energy at each pixel, etc. Note that this is merely an example, and predicted VMI patch 612 can include CT values associated with any suitable number of X-ray energies (e.g., one, two, three, etc.), and/or any suitable X-ray energy values (e.g., 40 keV, 50 keV, 130 keV etc.). In a more particular example, a first predicted VMI patch 612 can include a predicted CT number for a first X-ray energy at each pixel, a second predicted VMI patch 612 can include a predicted CT number for a second X-ray energy at each pixel, etc.

In some embodiments, predicted VMI patch(es) 612 can be compared to the corresponding ground truth MCT patch(es) 608 to evaluate the performance of first untrained CNN 610. Additionally, in some embodiments, VMI patch(es) 612 and a CT patch(es) 614 can be provided as inputs to a pre-trained general image recognition CNN 616 (e.g., VGG-19). A first output (e.g., VMI patch features 618) and a second output (e.g., CT patch features 620) of a hidden layer of the pre-trained general image recognition CNN can also be compared using the loss function (e.g., $L(f_{CNN1})$) to evaluate the performance of first untrained CNN 610. In some embodiments, CT patch 614 can be a portion of a routine-dose CT image of a phantom corresponding to MECT patch 604. Additionally or alternatively, CT patch 614 can be a portion of an MECT image corresponding to a particular portion of X-ray energies. For example, CT patch 614 can be a $T_L$ image generated from PCD-CT data. As another example, CT patch 614 can be a low energy image generated form dual-energy CT data.

In some embodiments, a loss value can be calculated using loss function $L(f_{CNN1})$ described above in connection with EQ. (6), which can be used to evaluate the performance of first untrained CNN 610 in predicting VMIs of particular energy levels from the particular MECT patch 604. In some embodiments, the loss value can be used to adjust weights of first untrained CNN 610. For example, a loss calculation 622 can be performed (e.g., by computing device 110, by server 120, by VMI system 106) to generate a loss value that can represent a performance of first untrained CNN 610. The loss value generated by loss calculation 622 can be used to adjust weights of first untrained CNN 610.

In some embodiments, after training has converged (and the trained CNN performs adequately on the test data), first untrained CNN 610 with final weights can be used to implement as a first trained CNN 630. In some embodiments, first untrained CNN 630 can be used with larger images (e.g., standard-sized MECT images of 512x512 pixels per image) after training (e.g., as described above in connection with trained CNN 324).

In some embodiments, first trained CNN 630 can be used to generate a predicted VMI(s) 654 of unlabeled MECT images at one or more energy levels, which can be presented and/or stored (e.g., for use in diagnosing a patient depicted in the VMI). Additionally or alternatively, in some embodiments, predicted VMI 654 can be refined by a second trained CNN 660.

In some embodiments, a second CNN can be trained using MECT patches 632, which can include patches that are similar (or identical to) MECT patches 304 described above in connection with FIG. 3. Additionally, in some embodiments, MECT patches 632, can include patches of MECT images of living subjects (e.g., human subjects, animal subjects). In some embodiments, MECT patches 632 can exclude patches that are included in a set of MECT patches 604 that were used to train first trained CNN 630.

In some embodiments, MECT patches 632 can be extracted from MECT images generated using a two-energy-threshold data acquisition technique, such as a technique described above in connection with MECT patches 604.

In some embodiments, noise (e.g., a random noise parameter which can be added or subtracted from the original value(s)) can be added (e.g., by computing device 110, by server 120, by VMI system 106) to one or more pixels of MECT patches 632 (e.g., as described above in connection with MECT patches 604). In some embodiments, use of real and synthesized low-dose CT data (e.g., MECT patches with noise added) to train a second untrained CNN can cause the second trained CNN 660 to be more robust to image noise in unlabeled low-dose MECT data (e.g., MECT image data for which densities distributions are unknown and/or not provided to the first CNN and/or second CNN during training).

In some embodiments, MECT patches 632 can be provided to first trained CNN 630, which can output corresponding VMI patch(es) 634, which can represent a portion of a VMI at particular X-ray energy $E_m$ (e.g., in kiloelectronvolt (keV)).

In some embodiments, a training dataset can be formed (e.g., by computing device 110, by server 120, by VMI system 106) from MECT patches 632 paired with corresponding VMI patch(es) 634, and corresponding CT patch(es) (not shown), which can be provided as input to image recognition CNN 616 to generate CT patch features 646. In some embodiments, a portion of MECT patches 632 can be used as training data (e.g., a training set, which can be divided into a training set and a validation set), and another portion of MECT patches 632 can be used as test data (e.g., a test set), which can be used to evaluate the performance of a CNN after training is halted. For example, 3/5 of MECT patches 632 can be used as training data, and the other 2/5 can be reserved as test data. In some embodiments, MECT patches 632 can be selected for inclusion in the training data as a group (e.g., MECT patches 632 generated from the same MECT image can be included or excluded as a group).

In some embodiments, a second untrained CNN 640 can be trained (e.g., by computing device 110, by server 120, by VMI system 106) using VMI patches 634. In some embodiments, second untrained CNN 640 can have any suitable topology, such as a topology described below in connection with FIG. 8.

In some embodiments, second untrained CNN 640 can be trained using an Adam optimizer (e.g., based on an optimizer described in Kingma et al., "Adam: A Method for Stochastic Optimization," 2014). As shown in FIG. 6, a particular sample MECT patch 632 can be provided as input to first trained CNN 630, which can output predicted VMI patch (or patches) 632, which can be provided as input to second untrained CNN 640, which can output a refined VMI patch(es) 642. In some embodiments, refined VMI patch(es) 642 can be formatted in any suitable format, such as formats described above in connection with predicted VMI patch(es) 612.

In some embodiments, refined VMI patch(es) 642 can be compared to VMI patch(es) 634 to evaluate the performance of second untrained CNN 640. Additionally, in some embodiments, refined VMI patch(es) 642 and corresponding CT patch(es) can be provided as inputs to image recognition CNN 616. A first output (e.g., refined VMI patch features 644) and a second output (e.g., CT patch features 646) of a hidden layer of image recognition CNN 616 can also be compared using the second loss function (e.g., $L(f_{CNN2})$) to evaluate the performance of second untrained CNN 640. In some embodiments, a CT patch used to generate CT patch features 646 can be a portion of a routine-dose CT image of a phantom corresponding to MECT patch 632 or a routine-dose CT image of a subject corresponding to MECT patch 632 as described above in connection with CT patch 614.

In some embodiments, a loss value can be calculated using loss function $L(f_{CNN2})$ described above in connection with EQ. (6), which can be used to evaluate the performance of second untrained CNN 640 in predicting refined VMIs of particular energy levels from the particular MECT patch 632. In some embodiments, the loss value can be used to adjust weights of second untrained CNN 640. For example, a loss calculation 648 can be performed (e.g., by computing device 110, by server 120, by VMI system 106) to generate a loss value that can represent a performance of second untrained CNN 640. The loss value generated by loss calculation 648 can be used to adjust weights of second untrained CNN 640.

In some embodiments, after training has converged (and the trained CNN performs adequately on the test data), second untrained CNN 640 with final weights can be used to implement second trained CNN 660. In some embodiments, second untrained CNN 660 can be used with larger images (e.g., standard-sized MECT images of 512x512 pixels per image) (e.g., as described above in connection with trained CNN 324).

In some embodiments, first trained CNN 630 can be used to generate predicted VMI(s) 654 of unlabeled MECT images at one or more energy levels, and predicted VMI(s) 654 can be provided as input to second trained CNN 660. Second trained CNN 660 can output a refined VMI(s) 662 corresponding to unlabeled MECT image 652, which can be presented and/or stored (e.g., for use in diagnosing a patient depicted in the VMI). For example, refined VMI(s) 662 (and/or VMI(s) 654) can represent a different versions(s) of MECT image 652 that have been transformed to highlight aspects of the MECT image contributed by attenuation of X-rays at a particular energy. Such a transformed image can cause certain features of a subject's anatomy to be presented more clearly than in a conventional CT or MECT image.

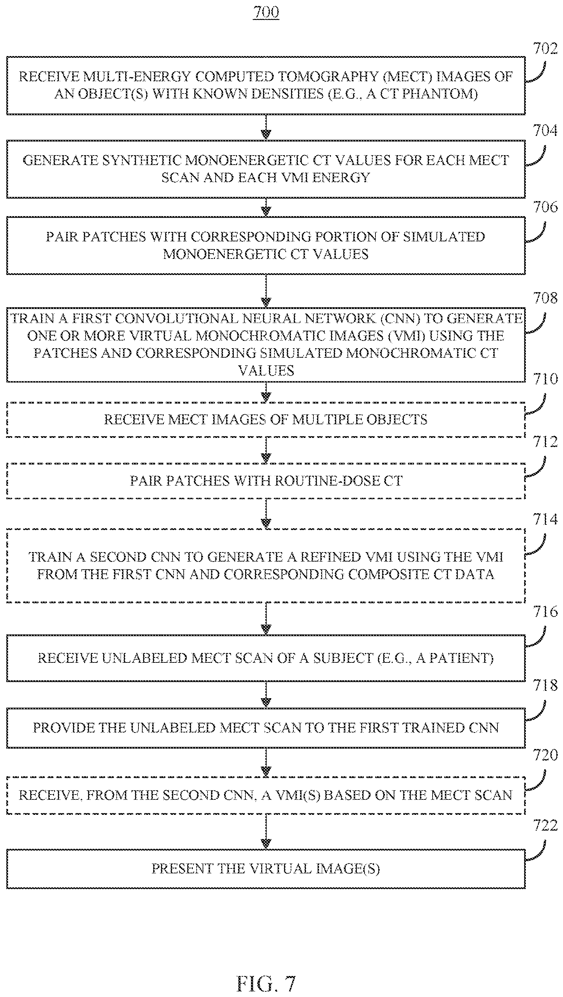

FIG. 7 shows an example of a process for training and using a convolutional neural network that can be used to implement mechanisms for virtual monoenergetic imaging from multi-energy computed tomography data in accordance with some embodiments of the disclosed subject matter. As shown in FIG. 7, process 700 can receive MECT images of an object with known materials having known mass densities at 702. For example, process 700 can receive MECT images of a phantom with known material properties. In a more particular example, process 700 can receive MECT images of one or more phantoms 601.

At 704, process 700 can generate one or more synthetic MCT images for each MECT image received at 702 based on the known material properties of the object(s) included in the MECT images, and for each X-ray energy level of interest (e.g., at 18 keV, 40 keV, at 50 keV, etc.). In some embodiments, synthetic MCT images can be formatted as a matrix and/or array of CT values (e.g., CT values corresponding to each pixel of a particular MECT image or set of images received at 702). For example, synthetic MCT image generated at 704 can correspond to a particular X-ray energy level, and can be formatted as an $i \times j$ matrix, where the MECT image has a size of $i \times j$ pixels. As a more particular example, each synthetic MCT image generated at 704 can correspond to a X-ray energy, and can be formatted as a $512 \times 512$ matrix, where the MECT image has a size of $512 \times 512$ pixels. As another example, each synthetic MCT images generated at 704 can represent multiple X-ray energy levels (e.g., all energy levels of interest), and can be formatted as an $i \times j \times m$ matrix, where the MECT image has a size of $i \times j$ pixels and there are m energy levels of interest.

At 706, process 700 can pair MECT patches (e.g., extracted and/or generated as described above in connection with 406 and/or 408 of FIG. 4) with a corresponding portion(s) of the synthetic MCT images generated at 704. For example, process 700 can determine which MECT image the patch was derived from, and which portion of MECT image the patch represents. In such an example, process 700 can generate a material mass density mask(s) that have the same dimensions (e.g., same height and width) as the patch.

At 708, process 700 can train a first CNN to predict one or more VMIs, each at a particular X-ray energy, from MECT images using the patches described above in connection with 706 as inputs, and using the synthetic MCT images paired with each patch at 706 to evaluate performance of the first CNN during training (and/or during testing). In some embodiments, process 700 can use any suitable technique or combination of techniques to train the CNN, such as techniques described above in connection with FIG. 6.

At 710, process 700 can receive additional MECT scans of one or more objects. For example, as described above in connection with MECT patches 632, process 700 can receive MECT images of one or more phantoms and/or one or more subjects (e.g., humans, animals, etc.).

At 712, process 700 can pair patches of the MECT images received at 710 with patches of routine-dose CT images of the same subject. For example, as described above in connection with CT patches 614 of FIG. 6, the routine-dose MECT image can be generated from a portion of the MECT images received at 710 (e.g., a $T_L$ image).

At 714, process 700 can train a second CNN to generate one or more refined VMIs, each at a particular X-ray energy, from VMI patches output by the first trained CNN as inputs, and using the VMI patch, and the CT patch to evaluate performance of the second CNN during training (and/or during testing). In some embodiments, process 700 can use any suitable technique or combination of techniques to train the second CNN, such as techniques described above in connection with FIG. 6.

At 716, process 700 can receive an unlabeled MECT image of a subject (e.g., a patient). In some embodiments, the unlabeled MECT image can be received from any suitable source, such as a CT machine (e.g., MECT source 102), a computing device (e.g., computing device 110), a server (e.g., server 120), and/or from any other suitable source.

At 718, process 700 can provide the unlabeled MECT image as input to the first trained CNN, which can generate a predicted VMI (or multiple predicted VMIs) at particular X-ray energy. The output of the first trained CNN can be provided as input to the second trained CNN.

At 720, process 700 can receive, from the second trained CNN, an output that includes one or more predicted VMIs corresponding to the unlabeled MECT image at particular X-ray energies. For example, process 700 can receive an output formatted as described above in connection with refined VMI 662. In another example, process 700 can receive an output formatted as an $i \times j \times m$ matrix, where the MECT image has a size of $i \times j$ pixels and there are m energies of interest, and each element of the matrix represents a predicted CT value representing a contribution of X-rays at the particular energy. As described above in connection with FIG. 6, in some embodiments, process 700 can omit training and/or use of the second CNN. For example, in some embodiments, 710, 712, and 714 can be omitted, and the first CNN can be used to generate a VMI without the second CNN described above. In some such embodiments, process 700 can receive a VMI from the first CNN at 720 (e.g., if the second CNN is omitted).

At 722, process 700 can cause the virtual image(s) (e.g., the VMIs) received at 720 and/or the original MECT image received at 716 to be presented. For example, process 700 can cause the VMI(s) and/or original MECT image to be presented by a computing device (e.g., computing device 110). Additionally or alternatively, in some embodiments, process 700 can cause the VMI(s) received at 720, the original MECT image received at 716, and/or any other suitable information, to be recorded in memory (e.g., memory 210 of computing device 110, memory 220 of server 120, and/or memory 228 of MECT source).

FIG. 8 shows an example of a topology of a convolutional neural network that can be used to implement mechanisms for virtual monoenergetic imaging from multi-energy computed tomography data in accordance with some embodiments of the disclosed subject matter. As shown in FIG. 8, the first CNN can include the same layers as the CNN described above in connection with FIG. 5, and the second CNN can include the same layers as the CNN described above in connection with FIG. 5, but can include a residual connection between the input and output. However, the input and outputs of the CNNs shown in FIG. 8 are different than the inputs and outputs of the CNN shown in FIG. 5.

FIG. 9 shows examples of virtual images generated using material maps derived from multi-energy computed tomography data of a porcine abdomen using mechanisms for virtual monoenergetic imaging described herein, and using other techniques, which can be used to generate a virtual monoenergetic image at any suitable energy. FIG. 9 shows various virtual images generated using techniques described herein (labeled with CNN-MD), and virtual images generated using other techniques. The first column includes virtual images showing the spatial distribution of hydroxyapatite (HA) in a CT image of the porcine abdomen, the second column includes virtual images showing the spatial distribution of iodine in the CT image, and the third column includes virtual images showing the spatial distribution of soft tissue in the CT image. As described above in connection with FIGS. 3 and 4, material maps generated using a CNN (e.g., as described above in connection with FIG. 3) can be used to generate virtual monoenergetic images at any suitable energy level.

The first row includes virtual images generated using techniques described herein. For example, as described above in connection with FIG. 3, predicted material mass densities were used to generate images that included only contributions from particular materials, or excluding certain materials. The second row includes virtual images generated using a total-variation material decomposition (TV-MD) technique, and the third row includes virtual images generated using a least squares material decomposition (LS-MD) technique. Note that all techniques compared in FIG. 9 are performed in the post-reconstruction domain, and are generated based on the same initial MECT images. The arrows in the iodine insets indicate the location of blood vessels. The arrows in the soft-tissue insets indicate the boundary of the kidney.

As shown in FIG. 9, the virtual images generated using techniques described herein preserve more detail, and generally include less noise than the virtual images generated using the other techniques. For example, using mechanisms described herein for facilitated improved performance on the animal datasets, for example, by providing improved visualization of small blood vessels and suppressing the image noise, including false enhancement of adipose tissue in the iodine image that was present in the LS-MD image. Additionally, the virtual images generated using techniques described herein exhibited improved quantitative accuracy (e.g., as shown in FIG. 10). The various techniques exhibited mean-absolute-error (MAE) of 0.61 (CNN-MD), 1.20 (TV-MD), and 1.36 (LS-MD) mgI/cc, for the iodine images.

FIG. 10 shows examples of predicted iodine concentration predicted from multi-energy computed tomography data of a phantom using mechanisms for virtual monoenergetic imaging described herein, and using other techniques. FIG. 10 includes predicted iodine concentration measured on iodine inserts (with 3, 6, 12, 18, 24 mgI/cc) with routine and low radiation dosed levels, using techniques described herein, a TS-MD technique, and an LS-MD technique. The left chart includes predicted concentrations, with error bars indicating the standard deviation of the estimated concentration. In the left chart the MECT images used to make the predictions were generated using a routine dose level (e.g., RD: $CTDI_{vol}$ 13 mGy). In the left chart the MECT images used to make the predictions were generated using a low dose level (e.g., LD: $CTDI_{vol}$ 7 mGy). Note that the x-axis represents the ground truth concentration, and the y-axis represents the predicted concentration. Accordingly, for the 3 mgI/cc concentration, an error can be calculated by determining a difference between the predicted value and 3 mgI/cc.

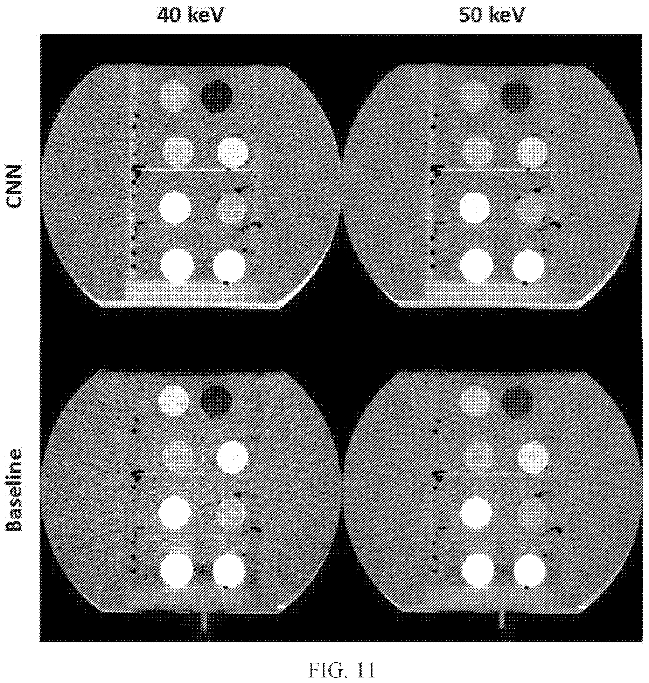

FIG. 11 shows examples of virtual monoenergetic images (VMIs) generated from multi-energy computed tomography data of a phantom using mechanisms for virtual monoenergetic imaging described herein, and using other techniques. As shown in FIG. 11, the 40 keV and 50 keV VMIs generated using techniques described herein (labeled as CNN) recovered more detail (e.g., the plastic stand at the bottom of the CNN images), and eliminated beam hardening artifacts highlighted by the arrows in the baseline images.

The first row includes VMIs generated using techniques described herein. For example, as described above in connection with FIG. 6, a VMI was directly generated by a first trained CNN from an MECT image of the phantom, and refined using a second trained CNN. The second row includes VMIs generated using commercial VMI software.

FIG. 12 shows examples of virtual monoenergetic images generated from multi-energy computed tomography data of a human subject using mechanisms for virtual monoenergetic imaging described herein, and using other techniques. As shown in FIG. 12, the 40 keV and 50 keV VMIs generated using techniques described herein (labeled as CNN) reduced noise compared to the baseline VMIs. Image noise was measured at the circular region-of-interests (ROIs) and is displayed next to each ROI. The VMIs generated using techniques described herein yielded ~50% and ~40% noise reduction at 40 keV and 50 keV, respectively (e.g., in Hounsfield Units).

The first row includes VMIs generated using techniques described herein. For example, as described above in connection with FIG. 6, a VMI was directly generated by a first trained CNN from an MECT image of the phantom, and refined using a second trained CNN. The second row includes VMIs generated using commercial VMI software.

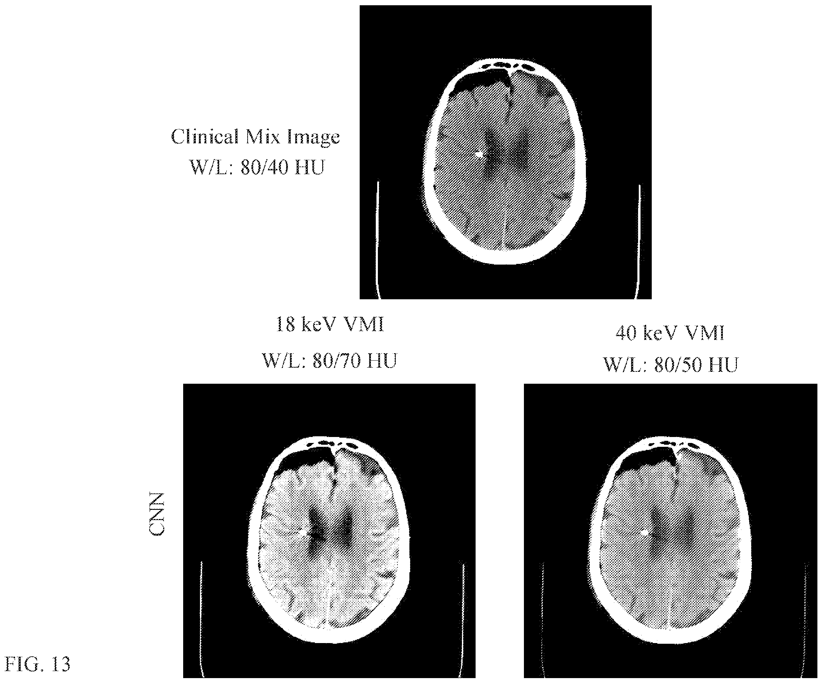

FIG. 13 shows examples of virtual monoenergetic images generated from multi-energy computed tomography data of a human subject using mechanisms for virtual monoenergetic imaging described herein, and a clinical mix image generated by a computed tomography scanner. The clinical mix image in FIG. 13 is a clinical dual-energy non-contrast head CT exam representing a type of image that is generally used in clinical practice. FIG. 13 also shows 18 keV and 40 keV VMIs generated using techniques described herein (labeled as CNN) using a single CNN (e.g., using a CNN trained as described above in connection with first trained CNN 630).

The clinical mix image lacks gray-white matter differentiation, while the VMIs generated using techniques described herein improves gray-white matter differentiation with both 18 keV and 40 keV images without magnifying image noise. As shown in FIG. 13, the 18 keV image exhibits increased differentiation between gray matter and white matter, with improved contrast-noise-ratio. The display window, specified using window width and level (W/L), was separately adjusted for the three different images to illustrate the increased differentiation in the lower X-ray energy VMIs.

VMI synthesized at ultra-low X-ray energy levels (e.g., on the order of less than 40 keV) can provide unprecedented differentiation of the soft-tissue without iodine-enhancement. For example, compared to conventional CT, 18 keV VMI can enhance gray-white-matter contrast in images of brain tissue, which can be leveraged in variety of neuroimaging applications. However, the image quality and quantitative accuracy of low-energy VMI from commercially available software suffers from significant noise amplification and beam hardening artifacts that impede clinical usefulness of low-energy VMI. For example, the quality of commercial VMI rapidly deteriorates as X-ray energy is decreased. Additionally, commercially available software does not provide ultra-low-energy VMI. Note that although an 18 keV VMI is shown in FIG. 13, mechanisms for virtual monoenergetic imaging described herein can be trained to generate one or more VMIs at any suitable ultra-low X-ray energy levels (e.g., at any suitable X-ray energy level below 40 keV).

Existing commercial software is typically based on standard basis material-decomposition algorithms that directly apply a pre-calibrated linear system model to patient CT images. Such pre-calibrated linear system model do not adequately model non-linear physical processes involved in the data acquisition of real CT systems. Additionally, standard basis material-decomposition techniques intrinsically amplify noise and artifacts that are already presented in the input multi-energy CT images. As described above in connection with FIGS. 6-8, mechanisms described herein can use customized CNN, objective function, and training techniques to generate ultra-low energy VMIs. For example, a CNN can be trained with CT images of standard CT phantom materials that are widely-accepted as good surrogates of real human tissues, and noise/artifact-free labels can be generated (e.g., based on the theoretical X-ray attenuation of the phantom materials) and used to train the CNN. This can cause the CNN to learn the direct functional mapping between polyenergetic dual-energy CT (e.g., MECT images) and monoenergetic CT. Unlike conventional techniques referenced above, a CNN trained in accordance with mechanisms described herein can implicitly learn underlying non-linear physical processes during training. Additionally, such training also facilitates suppression by the CNN of noise and artifacts that can be propagated from the input MECT images. Additionally, techniques described herein can utilize exclusively phantom data (e.g., patient CTs may be unnecessary) for training. Accordingly, a CNN can be trained using a scanner that is not commercially available and/or not yet approved for use with human subjects.

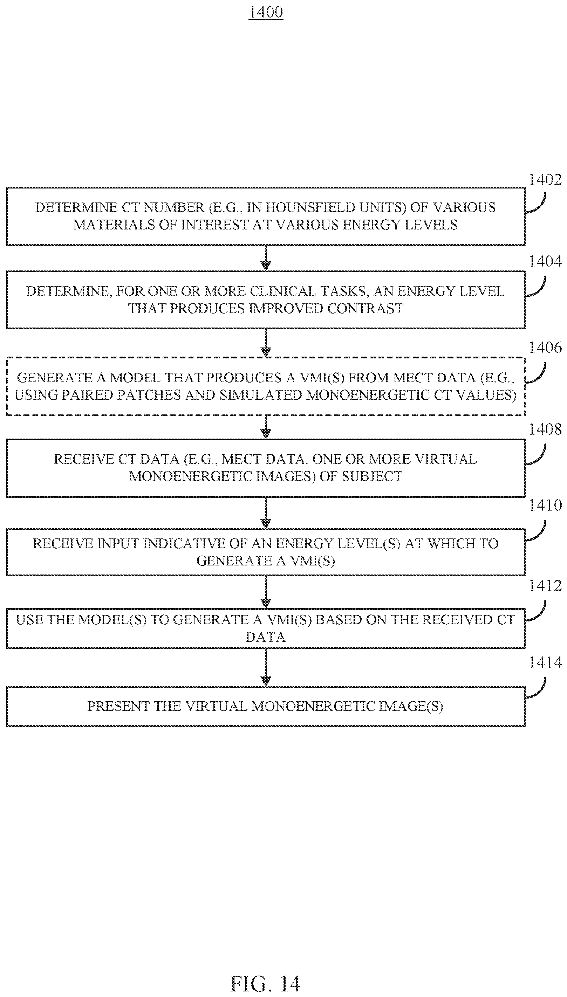

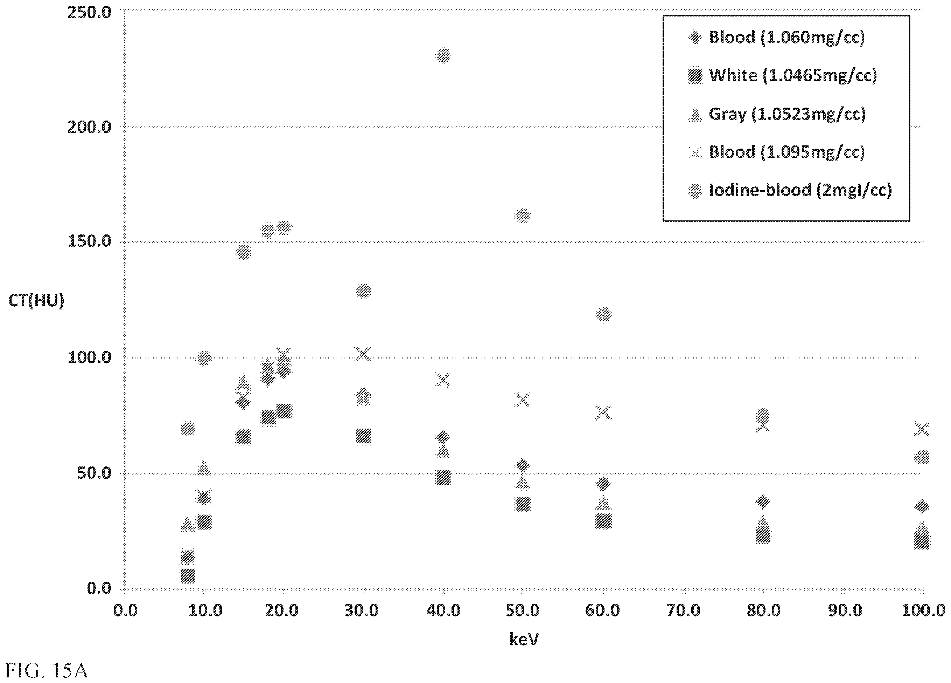

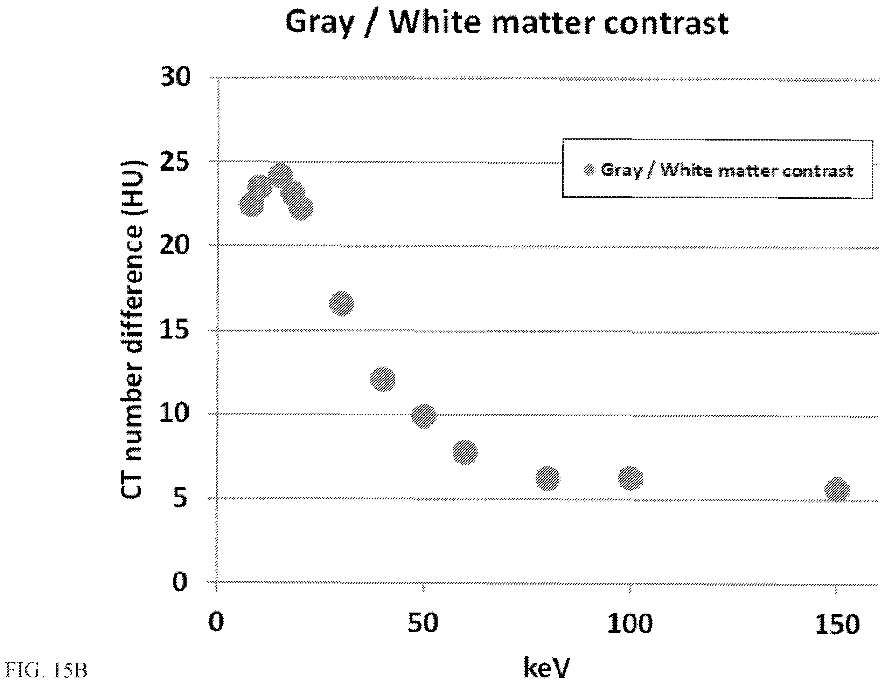

FIG. 14 shows an example 1400 of a process for generating computed tomography (CT) images with improved contrast in accordance with some embodiments of the disclosed subject matter. As shown in FIG. 14, process 1500 can include determining an attenuation coefficient of multiple target materials at various X-ray energies. As described below in connection with FIGS. 15A and 15B, the amount by which a material attenuates X-rays can vary with X-ray energy. In some embodiments, any suitable metric can be used to quantify the attenuation coefficient of the materials. For example, process 1400 can include determining a CT number (e.g., in Hounsfield units) associated with each material at a particular density of the material(s).

In some embodiments, any suitable technique or combination of techniques can be used to determine an attenuation coefficient associated with each material. For example, attenuation associated with various materials can be analytically calculated based on properties of the materials. In a more particular example, process 1400 can include using theoretically and/or empirically derived mass attenuation coefficients (e.g., in units of centimeters squared per gram ($cm^2/g$)). Such coefficients can be obtained using the XCOM database made available by the National Institutes of Standards and Technology Physical Measurement Laboratory (e.g., available via physics(dot)nist(dot)gov/physrefdata/xcom/html/xcom1html). In such an example, an attenuation coefficient $\mu$ at a particular X-ray energy can be calculated by multiplying the mass attenuation coefficient by the mass density, and a CT number in Hounsfield units can be calculated using the relationship: $$HU = 1000 \times \frac{\mu - \mu_{water}}{\mu_{water} - \mu_{air}}$$ . In another more particular example, process 1400 can include using information provided by a manufacturer and/or vendor of a surrogate material that closely mimics a target material (e.g., phantom materials that are considered to be equivalent to the basis materials that form various human tissues or organs) to determine mass attenuation coefficients, attenuation coefficients, and/or CT numbers associated with the various target materials at different X-ray energies. Due to very similar X-ray attenuation characteristics, such phantom materials can be considered to be equivalent to basis materials that form various human tissues and/or organs. FIGS. 3 and 12 include CT images of phantoms.

In some embodiments, process 1400 can include identifying target materials using any suitable technique or combination of techniques. For example, process 1400 can include identifying materials that are likely to be included in a CT scan associated with a particular task. In a more particular example, a brain CT exam with an iodine-based contrast-agent can generate CT image data that represents brain tissue (e.g., grey matter and white matter), iodine, a mixture of iodine and blood, bone (e.g., hydroxyapatite), and fatty tissue (e.g., adipose tissue). In another more particular example, an iodine-enhanced chest CT can generate CT image data that represents lung tissue, iodine, a mixture of iodine and blood, bone (e.g., hydroxyapatite), and fatty tissue (e.g., adipose tissue).

In some embodiments, process 1400 can plot the attenuation coefficient (e.g., represented in HU) for each material across a range of X-ray energies. For example, as described below in connection with FIG. 15A, process 1400 can plot points and/or a curve representing the attenuation coefficients of the materials across a range of X-ray energies and/or a. Additionally or alternatively, process 1400 can generate and/or record functions that represent the attenuation coefficients of the materials across a range of X-ray energies.

At 1404, process 1400 can determine, for various clinical tasks, one or more energy levels that produce improved contrast between target materials. For example, process 1400 can determine an X-ray energy (or energies) from within a particular range of X-ray energies, that can improve contrast between the target materials when compared to contrast between the target materials in an MECT image (e.g., corresponding to a weighted average of components from X-ray sources) or a monoenergetic image (e.g., a VMI) at a particular energy level. As another example, process 1400 can determine an X-ray energy (or energies) from within a particular range of X-ray energies, that can maximize contrast between the target materials. As yet another example, process 1400 can determine an X-ray energy (or energies) from within a particular range of X-ray energies, that can provide a best contrast among the target materials.

In some embodiments, process 1400 can include identifying an X-ray energy level within a predetermined range of X-ray energies that provides the largest difference in attenuation coefficient between two or more target materials associated with a particular task. For example, process 1400 can identify X-ray energies in a range of about 8-20 keV from within a range of about 1 keV to about 150 keV as providing improved contrast between gray matter and white matter for a brain scan. In a more particular example, process 1400 can identify X-ray energies of about 18 keV from within a range of about 1 keV to about 150 keV as providing a largest contrast between gray matter and white matter for a brain scan to be analyzed for a clinical task focused on identifying gray matter and/or white matter in the brain.

As another example, process 1400 can identify X-ray energies in a range of about 14-55 keV from within a range of about 1 keV to about 150 keV as providing improved contrast between brain tissue (e.g., gray matter and white matter) and a mixture of iodine and blood for a brain scan captured with an iodine-based contrast agent to be analyzed for a clinical task focused on identifying blood flow in the brain.

As yet another example, process 1400 can identify X-ray energies in a range of about 33-55 keV from within a range of about 1 keV to about 150 keV as providing improved contrast between normal liver tissue and abnormal tissue in the liver (e.g., a tumor) for an abdominal scan (e.g., captured with an iodine-based contrast agent) to be analyzed for a clinical task focused on identifying the presence of a tumor in the liver. In such an example, a tumor can be associated with higher concentrations of blood and iodine than normal tissue, as the tumor may have a more robust blood supply, and process 1400 can identify X-ray energies that provide improved contrast between liver tissue, blood, and/or a mixture of blood and iodine.

As still another example, process 1400 can identify X-ray energies in a range of about 15-50 keV from within a range of about 1 keV to about 150 keV as providing improved contrast between benign and/or malignant tumor tissues and tumor tissues changes with response to treatment (e.g., scar tissue, fibrosis) for a scan to monitor the status of a tumor (e.g., during a course of treatment). In such an example, a VMI with improved contrast can be analyzed to more accurately determine a likelihood that a tumor is responding to treatment, as change of tissue in a tumor may be indicative of response to treatment, in addition to, or in lieu of, the size of the tumor. The size of a tumor may not correlate with successful treatment and/or may be a lagging indicator of success (e.g., a tumor that is responding to treatment may develop scar tissue before the tumor's size is reduced). In such an example, process 1400 can identify X-ray energies that provide improved contrast between blood and/or a mixture of blood and iodine (e.g., corresponding to undamaged tumor tissue with robust blood supply), and different tissues (e.g. normal, fibrosis, scar, benign or malignant tumors).

In some embodiments, process 1400 can consider a smaller range of energy levels when determining an energy that produces improved contrast. For example, a CNN-based technique used to generate a VMI (e.g., as described above in connection with FIGS. 3 and/or 6) can be trained to produce a VMI(s) at a particular energy level(s) within a relatively large range of energy levels without degrading the signal to noise ratio (SNR) below an acceptable level. As another example, some techniques used to generate VMIs (e.g., a least squares regression technique) may amplify noise within a certain range of X-ray energies (e.g., at low X-ray energies), and a range from which an X-ray energy can be selected by process 1400 can be reduced to a range in which a technique used to generate a VMI produces an image with a sufficiently high SNR to be useful. Below a particular energy level, VMIs produced via the least-squares technique may produce images with relatively low SNR.

In a particular example, when a least squares approach is used to generate a VMI, process 1400 can identify an X-ray energy level from within a range of X-ray energy levels with acceptable noise levels (e.g., about 40 keV to about 150 keV), and can identify an X-ray energy (e.g., of about 40 keV) as providing improved contrast between gray matter and white matter for a brain scan.

At 1406, process 1400 can generate a model that generates one or more VMIs, each at a particular X-ray energy or energies, using patches from an MECT image of one or more phantoms (e.g., such as MECT patches described above in connection with 604) and synthetic MCT images of the one or more phantoms (e.g., as described above in connection with 608) and/or other data related to the one or more phantoms (e.g., material mass densities of the phantoms, as described above in connection with 308). Alternatively, in some embodiments, if a CT scanner generates one or more monoenergetic images (e.g., VMIs) as output (e.g., in addition to, or in lieu of, an MECT image). For example, the CT scanner may use an unknown (e.g., proprietary) technique to generate a VMI at a particular energy, and mechanisms described herein can use such a VMI as training data to generate a morel that predicts a VMI at a different energy. In some embodiments, 1406 can be omitted. For example, if an existing model is to be used to generate a VMI(s), 1406 can be omitted.

In some embodiments, process 1400 can generate any suitable model at 1406 (e.g., a statistical model, a machine learning model) using any suitable technique or combination of techniques (e.g., one or more statistical modeling techniques, one or more machine learning modeling techniques). For example, process 1400 can use techniques described above in connection with FIG. 6 to train a CNN to predict one or more VMIs from MECT image data. As another example, process 1400 can use one or more least squares techniques to generate a trained model to predict one or more VMIs from MECT image data. In such an example, measured CT numbers (e.g., from an MECT or VMI image generated by a CT scanner) and CT numbers from the synthetic MCT image can be used to establish a regression model(s) that can be used to estimate VMI CT numbers for the same target materials represented by the phantom(s) at different mass densities. In such an example, a least squares technique can be used to generate a trained model that can be used to derive material mass densities from measured CT numbers (e.g., from an MECT or VMI image generated by a CT scanner), and the material mass densities can be used to estimate a VMI of a particular energy. As yet another example, iterative-based techniques, such as prior-knowledge-aware iterative denoising (e.g., described in Tao et al., "Improving iodine contrast to noise ratio using virtual monoenergetic imaging and prior-knowledge-aware iterative denoising (mono-PKAID)," Phys Med Biol (2019)), can be used to implement a trained model that can be used to estimate a VMI of a particular energy from MECT data.

In some embodiments, process 1400 can generate a model based on projection domain CT data. For example, in addition, or in lieu, of using CT image data output by a CT scanner, process 1400 can use projection domain data prior from which CT image data is generated to generate a model.

At 1408, process 1400 can receive CT data of a subject from a CT source. In some embodiments, the CT data can be output by the CT source in any suitable format. For example, the CT source can be an MECT source (e.g., MECT source 102) that outputs MECT data. As another example, the CT source can directly output a VMI(s) at a particular energy level or energy levels (e.g., the CT source can generate a VMI using projection domain data).

At 1410, process 1400 can receive input indicative of an energy level or energy levels at which one or more VMIs are to be generated. In some embodiments, the input can be received in any suitable format and/or using any suitable technique. For example, process 1400 can receive input explicitly indicating one or more energy levels at which process 1400 is to generate VMIs. In such an example, process 1400 can receive input via a user input device (e.g., via inputs 206) reflecting a particular energy level or energy levels at which to generate a VMI(s). In a more particular example, a user of a virtual monoenergetive imaging system (e.g., VMI system 106) may provide input indicating that an 18 keV VMI (and/or a VMI(s) of any other energy) is to be generated.

As another example, process 1400 can receive input implicitly indicating one or more energy levels at which process 1400 is to generate VMIs. In such an example, process 1400 can receive information indicative of a task(s) for which the VMI(s) is to be generated, and process 1400 can automatically determine one or more energy levels at which VMIs are to be generated. Such information can include an explicit selection of a task to be performed (e.g., provided via a user input device, such as inputs 206; provided from an electronic medical record system, such as via a code associated with an order for a CT scan; and/or via any other suitable mechanism), or an explicit selection of materials of interest.

Additionally or alternatively, in some embodiments, process 1400 can automatically predict a task based on image data received from a CT source (e.g., multi-energy CT source 102). For example, process 1400 can analyze an MECT image and/or one or more VMIs, and can predict one or more target materials included in the image. In such an example, process 1400 can automatically (e.g., without user intervention) identify one or more VMIs to generate based on the prediction of the task being performed.

Additionally or alternatively, in some embodiments, process 1400 can generate a VMI at multiple energy levels, and process 1400 can determine based on input at 1410 which of the generated VMIs is to be presented.

In some embodiments, process 1400 can receive input indicative of an energy level(s) at which to generate a VMI(s) at 1410 after CT data is received from the CT source (e.g., any time subsequent to generation of CT data by the CT source). Alternatively, in some embodiments, process 1400 can receive input indicative of an energy level(s) at which to generate a VMI(s) at 1410 before CT data is generated or received (e.g., prior to a CT procedure). In such embodiments, 1410 can precede 1408. For example, input can be received at 1410 prior to a CT image being generated by a CT source, and the CT source can generate a VMI (e.g., based on MECT image data, based on MECT projection domain data, etc.) at one or more energy levels indicated by the input.

At 1412, process 1400 can use the model(s) generated at 1406 (and/or another model) to generate a VMI(s) at one or more particular energy levels using the received CT data using any suitable technique or combination of techniques. For example, process 1400 can use a CNN trained to generate a VMI(s) at a particular energy level to generate the VMI(s) at the one or more energy levels indicated by input received at 1410. As another example, process 1400 can use a least squares model to generate the one or more VMI(s).

At 1414, process 1400 can cause the VMI(s) generated at 1412 and/or original CT data received at 1408 to be presented. For example, process 1400 can cause the VMI(s) and/or original CT image to be presented by a computing device (e.g., computing device 110). Additionally or alternatively, in some embodiments, process 1400 can cause the VMI(s) generated at 1412, an original CT image received at 1408, and/or any other suitable information, to be recorded in memory (e.g., memory 210 of computing device 110, memory 220 of server 120, and/or memory 228 of MECT source).

FIG. 15A shows examples of calculated monoenergetic CT numbers for various materials across a range of X-ray energies. For example, CT numbers (in Hounsfield units) for gray matter, white matter, blood (at two different densities), and a mixture of iodine and blood (e.g. iodine-enhanced blood) are plotted for various X-ray energies in a range of 8 keV to 100 keV. As shown in FIG. 15A, the maximal contrast in this range of energies between gray matter and white matter (e.g., a difference of about 22-24 HU) is achieved at an X-ray energy of about 18 keV to 20 keV, and the contrast is slightly lower at lower energies (e.g., in a range of about 8 keV to about 15 keV). The contrast between white matter and gray matter generally decreases as X-ray energy increases (e.g., at 100 keV there is very little contrast between gray matter and white matter). FIG. 15B shows an example of contrast between gray matter and white matter across a range of x-ray energies. As shown in FIG. 15B, contrast between gray and white matter in the brain is maximized at around 18 keV.

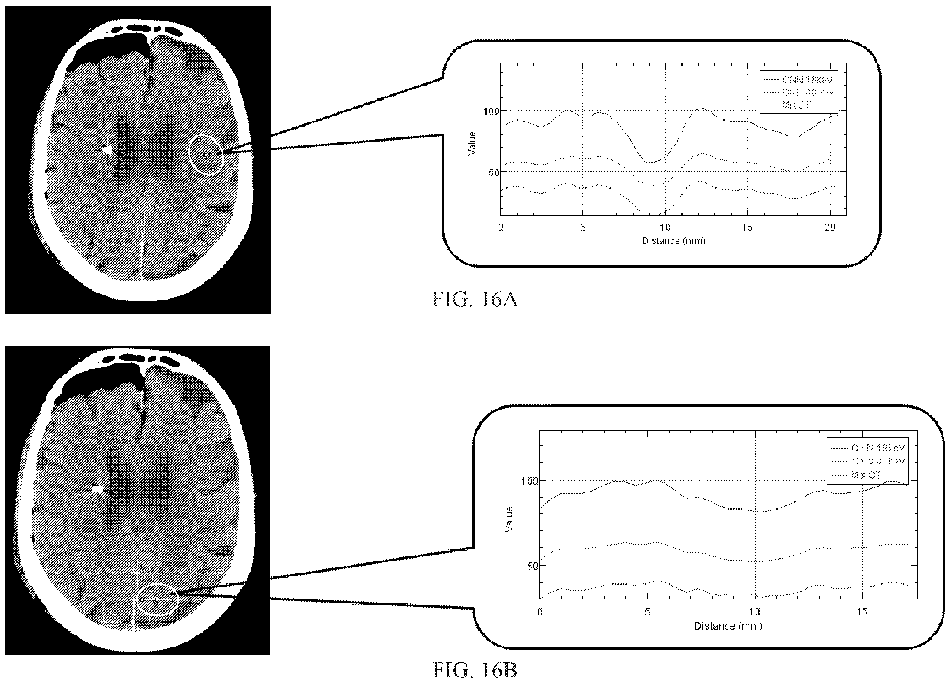

FIGS. 16A and 16B show examples of line profiles for the clinical mix image, and virtual monoenergetic images shown in FIG. 13 at two positions. The positions of the line profiles are illustrated with a line, and an ellipse is shown around the line to highlight the location of the line. As shown in FIG. 16A, the 18 keV VMI generated using mechanisms for virtual monoenergetic imaging described herein exhibits an increased range of CT values (e.g., from about 60-100 Hounsfield units, whereas the clinical mix image has a range from about 15-40 Hounsfield units), which can be observed at the transition between gray matter and white matter near 9 mm. In FIG. 16B, an increased range of CT values is apparent in the 18 keV VMI generated using mechanisms for virtual monoenergetic imaging described herein.

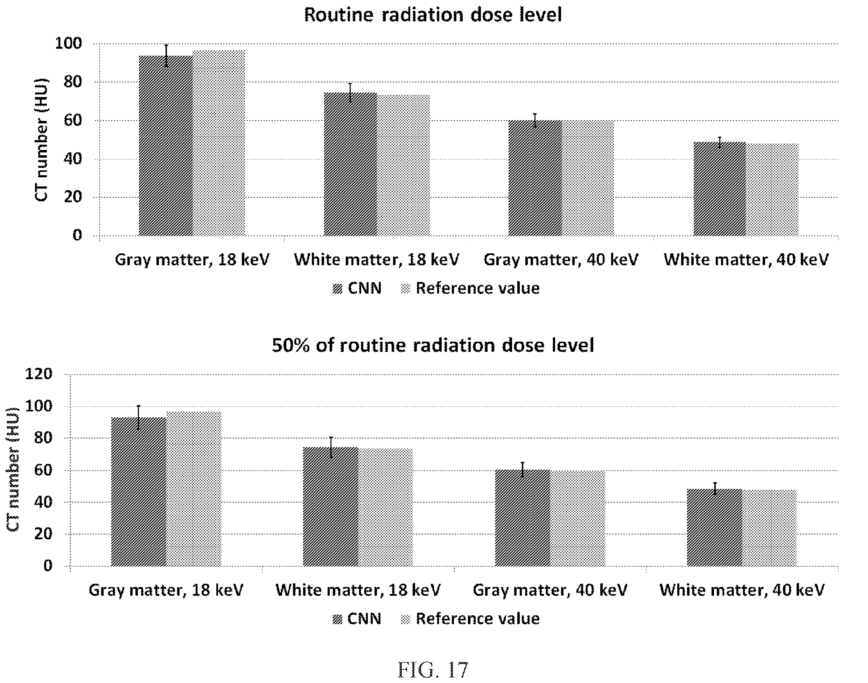

FIG. 17 shows examples of predicted CT number for virtual monoenergetic images generated from multi-energy computed tomography data of a phantom materials representing gray matter and white matter using mechanisms for virtual monoenergetic imaging described herein at two different radiation dose levels. FIG. 17 includes predicted CT number (in Hounsfield units) for gray matter and white matter for an 18 keV VMI and a 40 keV VMI generated using techniques described herein (labeled as CNN), and reference values generated using calculated CT values for a portion of the phantom representing gray matter and white matter if the phantom were imaged using a particular X-ray energy $E_m$ (e.g., in kiloelectronvolt (keV)). The VMIs were generated by a CNN trained in accordance with mechanisms described herein (e.g., using a CNN trained as described above in connection with first trained CNN 630) provided with MECT data of a phantom that includes materials that are widely-accepted as good surrogates for human brain tissue, including a portion representing gray matter and a portion representing white matter. Monoenergetic CT numbers used as reference values in FIG. 17 can include information indicative of an average attenuation at a portion of the phantom if the phantom were exposed exclusively to X-rays at the particular energy. As shown in FIG. 17, quantitative accuracy of materials of interest was maintained for VMIs at both energy levels, and both routine radiation dose and 50% of routine radiation dose (e.g., low-dose). The contrast-noise-ratio (CNR) was 1.6 for the clinical mix image, 2.8 for the 40 keV VMI, and 2.6 for the 18 keV VMI.

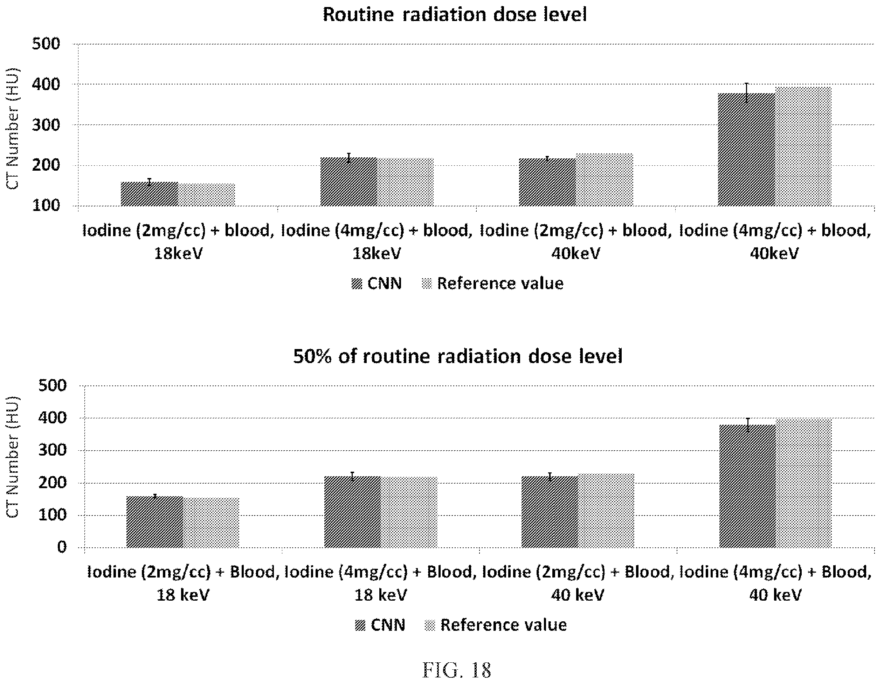

FIG. 18 shows examples of predicted CT number for virtual monoenergetic images generated from multi-energy computed tomography data of phantom materials representing mixtures of iodine and blood using mechanisms for virtual monoenergetic imaging described herein at two different radiation dose levels. FIG. 18 includes predicted CT number (in Hounsfield units) for a mixture of iodine and blood at different iodine concentrations (2 mg/cc and 4 mg/cc) for an 18 keV VMI and a 40 keV VMI generated using techniques described herein (labeled as CNN), and reference values generated using calculated CT values for portions of a phantom representing iodine at the different concentrations if the phantom were imaged using a particular X-ray energy $E_m$ (e.g., in keV). The VMIs were generated by a CNN trained in accordance with mechanisms described herein for virtual monoenergetic imaging (e.g., using a CNN trained as described above in connection with trained CNN 630) from MECT data of the phantom that includes materials that are widely-accepted as good surrogates for the materials of interest, including a portion representing blood and iodine at 2 mg/cc and a portion representing blood and iodine at 4 mg/cc. Reference values in FIG. 18 can include information indicative of the CT number of iodine represented by the portion of the phantom. As shown in FIG. 18, quantitative accuracy of materials of interest was maintained for both concentrations, and both routine radiation dose and 50% of routine radiation dose (e.g., low-dose).

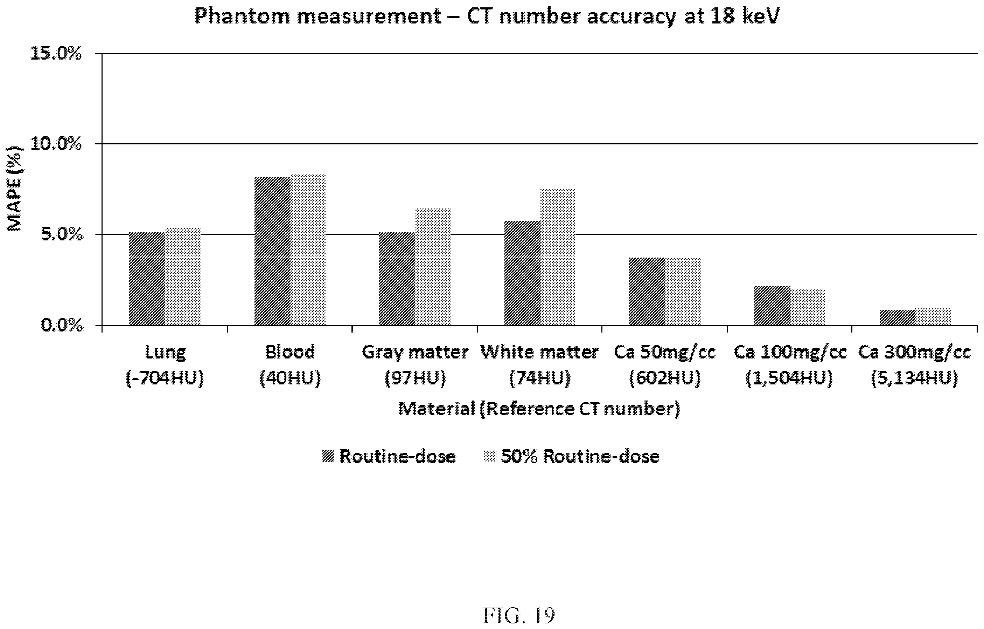

FIG. 19 shows examples of mean-absolute-percent-error (MAPE) in CT number of predicted CT number for virtual monoenergetic images generated from multi-energy computed tomography data of various materials. FIG. 19 includes the MAPE between a predicted CT number and a reference CT number for each material at 18 keV at a routine-dose and a 50% dose, including lung tissue (reference CT number -704 HU), blood (reference CT number 40 HU), gray matter (reference CT number 97 HU), white matter (reference CT number 74 HU), calcium at 50 mg/cc (reference CT number 602 HU), calcium at 100 mg/cc (reference CT number 1,504 HU), and calcium at 300 mg/cc (reference CT number 5,134 HU). The results in FIG. 19 were generated by a CNN trained in accordance with mechanisms described herein for virtual monoenergetic imaging (e.g., using a CNN trained as described above in connection with trained CNN 630) from MECT data of a phantom that includes materials that are widely-accepted as good surrogates for the materials of interest. As shown in FIG. 19, CT numbers generated using mechanisms described herein demonstrate consistent CT accuracy at different radiation dose levels, with a MAPE range from about 0.8 % to about 8.3 %.

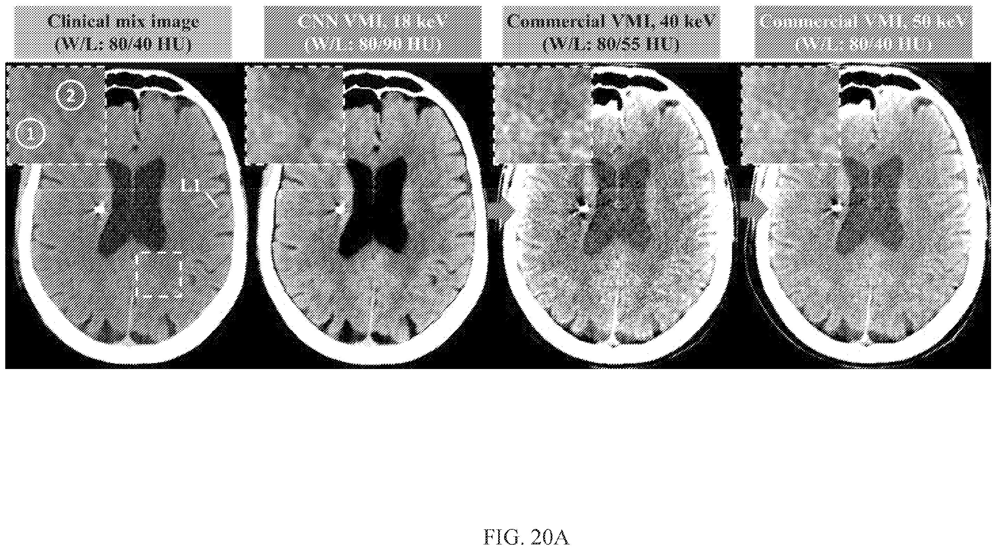

FIG. 20A shows examples of a virtual monoenergetic image generated from multi-energy computed tomography data of a human subject using mechanisms for virtual monoenergetic imaging described herein, a clinical mix image generated by a computed tomography scanner, and virtual monoenergetic images generated from multi-energy computed tomography data of the human subject using other techniques. The clinical mix image in FIG. 20A is a clinical dual-energy non-contrast head CT exam representing a type of image that is generally used in clinical practice. Each image is shown with an enlarged portion that includes two regions of interest labeled #1 and #2. FIG. 20A also shows an 18 keV VMI generated using techniques described herein (labeled as CNN VMI) using a single CNN (e.g., using a CNN trained as described above in connection with first trained CNN 630), and 40 keV and 50 keV VMIs generated using commercially available VMI software. Note that the 18 keV VMI image in FIG. 20A is the same image shown in FIG. 13 with a different width and level (W/L) (i.e., 80/90 rather than 80/70).

As shown in FIG. 20A, the clinical mix image lacks gray-white matter differentiation, and the 40 and 50 keV VMIs generated using the commercial software are noisy (e.g., have low contrast-noise-ratios) and include beam-hardening artifacts (shown with arrows). The 18 keV VMI generated using techniques described herein improves gray-white matter differentiation without magnifying image noise. As shown in FIG. 20A, the 18 keV image exhibits increased differentiation between gray matter and white matter, with improved contrast-noise-ratio. The display window, specified using window width and level (W/L), was separately adjusted for the three different images to illustrate the increased differentiation in the lower X-ray energy VMIs. The display window, specified using window width and level (W/L), was separately adjusted for the different images to illustrate the increased differentiation in the lower X-ray energy VMIs.

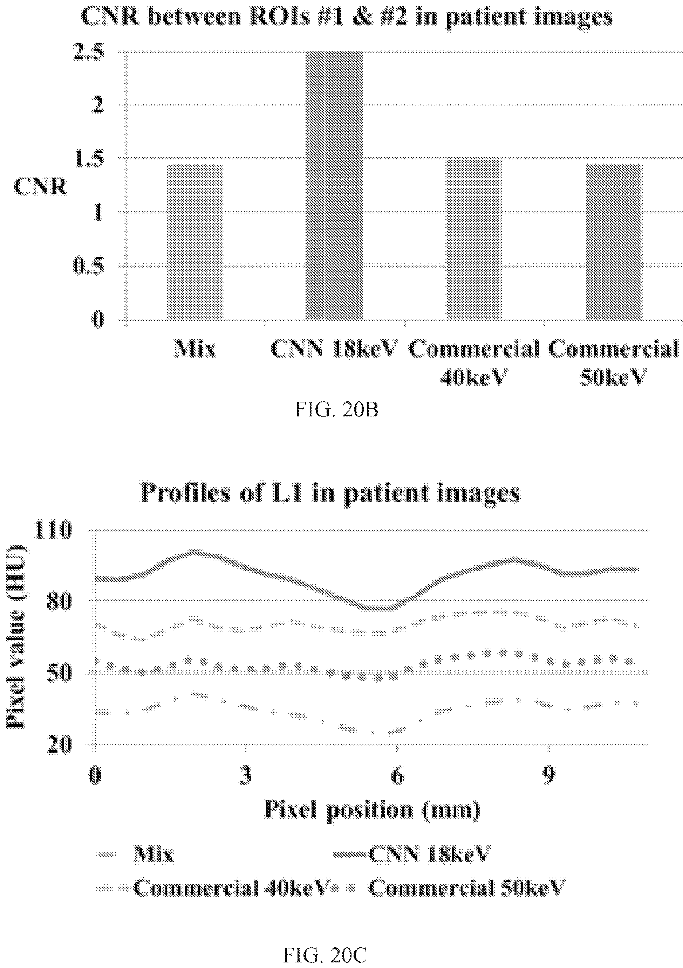

FIG. 20B shows contrast-to-noise ratio (CNR) between ROIs #1 and #2 in the images shown in FIG. 20A. As shown in FIG. 20B, the 18 keV image generated using mechanisms described herein had the highest CNR.

FIG. 20C an example of line profiles for the clinical mix image, and virtual monoenergetic images shown in FIG. 20A. As shown in FIG. 20C, the 18 keV VMI generated using mechanisms for virtual monoenergetic imaging described herein exhibits an increased range of CT values (e.g., from about 75-100 Hounsfield units, whereas the clinical mix image has a range from about 20-40 Hounsfield units), which can be observed at the transition between gray matter and white matter from 6-9 mm. In FIG. 20C, an increased range of CT values is apparent in the 18 keV VMI generated using mechanisms for virtual monoenergetic imaging described herein. The range of CT values in the VMIs generated using the commercial softrware also show limited range of CT values.

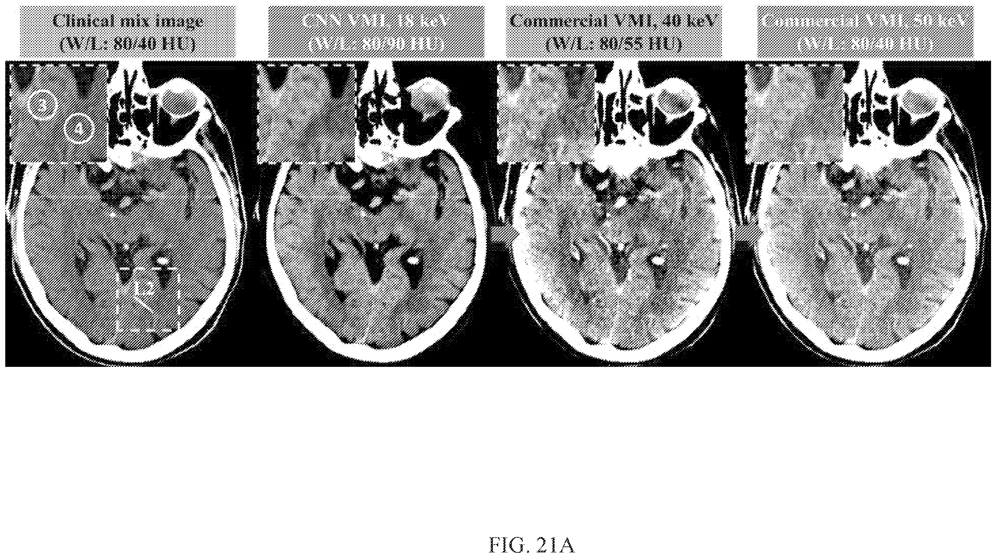

FIG. 21A shows additional examples of a virtual monoenergetic image generated from multi-energy computed tomography data of a human subject using mechanisms for virtual monoenergetic imaging described herein, a clinical mix image generated by a computed tomography scanner, and virtual monoenergetic images generated from multi-energy computed tomography data of the human subject using other techniques. The clinical mix image in FIG. 21A is a clinical dual-energy non-contrast head CT exam representing a type of image that is generally used in clinical practice. Each image is shown with an enlarged portion that includes two regions of interest labeled #3 and #4. FIG. 21A also shows an 18 keV VMI generated using techniques described herein (labeled as CNN VMI) using a single CNN (e.g., using a CNN trained as described above in connection with first trained CNN 630), and 40 keV and 50 keV VMIs generated using commercially available VMI software.

As shown in FIG. 21A, the clinical mix image lacks gray-white matter differentiation, and the 40 and 50 keV VMIs generated using the commercial software are noisy (e.g., have low contrast-noise-ratios) and include beam-hardening artifacts (shown with arrows). The 18 keV VMI generated using techniques described herein improves gray-white matter differentiation without magnifying image noise. As shown in FIG. 21A, the 18 keV image exhibits increased differentiation between gray matter and white matter, with improved contrast-noise-ratio.

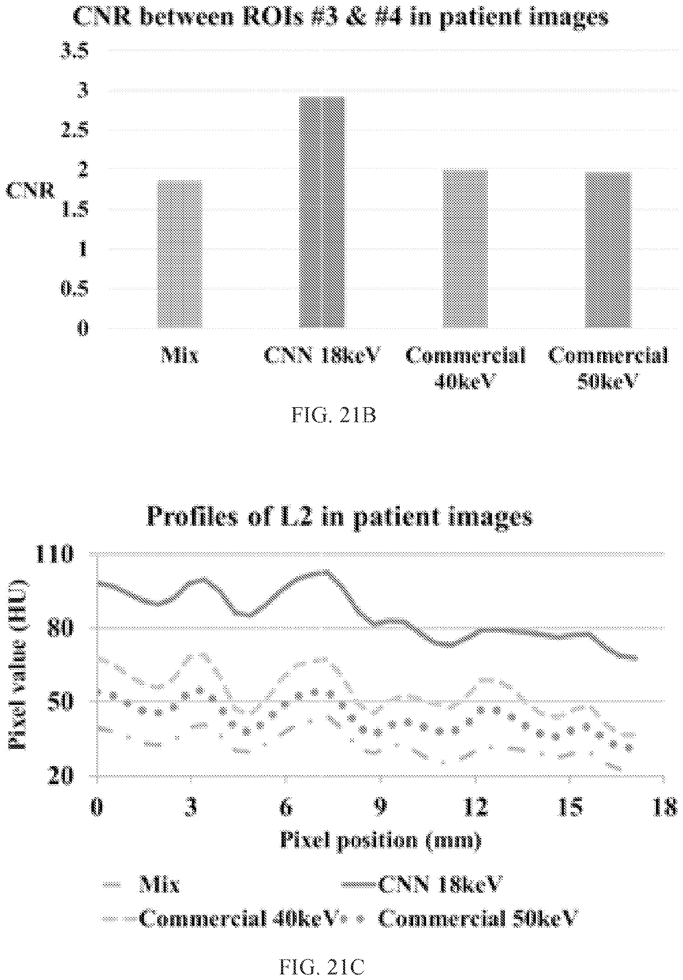

FIG. 21B shows contrast to noise ratio between two regions of interest in the images shown in FIG. 21A. As shown in FIG. 21B, the 18 keV image generated using mechanisms described herein had the highest CNR.

FIG. 21C an example of line profiles for the clinical mix image, and virtual monoenergetic images shown in FIG. 21A. As shown in FIG. 21C, the 18 keV VMI generated using mechanisms for virtual monoenergetic imaging described herein exhibits an increased range of CT values (e.g., from about 70-100 Hounsfield units)z, whereas the clinical mix image has a range from about 20-40 Hounsfield units). In FIG. 21C, an increased range of CT values is apparent in the 18 keV VMI generated using mechanisms for virtual monoenergetic imaging described herein. The range of CT values in the VMIs generated using the commercial softrware also show limited range of CT values.

In some embodiments, any suitable computer readable media can be used for storing instructions for performing the functions and/or processes described herein. For example, in some embodiments, computer readable media can be transitory or non-transitory. For example, non-transitory computer readable media can include media such as magnetic media (such as hard disks, floppy disks, etc.), optical media (such as compact discs, digital video discs, Blu-ray discs, etc.), semiconductor media (such as RAM, Flash memory, electrically programmable read only memory (EPROM), electrically erasable programmable read only memory (EEPROM), etc.), any suitable media that is not fleeting or devoid of any semblance of permanence during transmission, and/or any suitable tangible media. As another example, transitory computer readable media can include signals on networks, in wires, conductors, optical fibers, circuits, or any suitable media that is fleeting and devoid of any semblance of permanence during transmission, and/or any suitable intangible media.

It should be noted that, as used herein, the term mechanism can encompass hardware, software, firmware, or any suitable combination thereof.

It should be understood that the above described steps of the processes of FIGS. 4, 7, and/or 14 can be executed or performed in any order or sequence not limited to the order and sequence shown and described in the figures. Also, some of the above steps of the processes of FIGS. 4, 7, and/or 14 can be executed or performed substantially simultaneously where appropriate or in parallel to reduce latency and processing times.

Although the invention has been described and illustrated in the foregoing illustrative embodiments, it is understood that the present disclosure has been made only by way of example, and that numerous changes in the details of implementation of the invention can be made without departing from the scope of the invention, which is limited only by the claims that follow. Features of the disclosed embodiments can be combined and rearranged in various ways.

**Claims**

1. A system for transforming computed tomography data, the system comprising:
   at least one hardware processor configured to:

   receive input indicative of a particular X-ray energy level at which to generate a virtual monoenergetic image (VMI) of a subject that includes a plurality of target materials with improved contrast, wherein the input indicative of the particular X-ray energy level at which to generate the VMI of the subject comprises an input explicitly indicating the plurality of target materials;
   receive computed tomography (CT) data of a subject;
   identify the particular X-ray energy as an X-ray energy at which contrast between the plurality of target materials is maximized;
   generate a VMI version of the CT data at the particular X-ray energy by:

   providing the CT data to a trained model, wherein the trained model is a trained convolutional neural network (CNN) that was trained using CT data of one or more subjects, each representing at least one material of a plurality of materials, and using synthesized monoenergetic CT images of at least one of the one or more subjects based on X-ray attenuation of the plurality of materials;
   receiving output from the trained model;
   generating the VMI version of the CT data based on the output of the trained model; and

   cause the VMI version of the CT data to be presented.

2. The system of claim 1, wherein the CT data comprises multi-energy computed tomography (MECT) image data of the subject.

3. The system of claim 1, wherein the CT data comprises MECT projection domain data of the subject.

4. The system of claim 1, wherein the subject includes a phantom comprising a plurality of samples, each of the plurality of samples representing at least one material of a plurality of materials.

5. The system of claim 1, wherein the trained model is a least squares model.

6. The system of claim 5, wherein the CT data is MECT image data.

7. The system of claim 1, wherein the plurality of target materials comprises at least two of:

   gray matter;
   white matter;
   an iodine contrast-media;
   calcium;
   a mixture of blood and iodine;
   adipose tissue;
   hydroxyapatite;

lung;
liver;
muscle; or
blood.

**8.** The system of claim 1, wherein the at least one hardware processor is further configured to:
generate a second VMI version of the CT data at a second particular X-ray energy.

**9.** The system of claim 8, wherein the second VMI version of the CT data is a 50 keV VMI.

**10.** The system of claim 8, wherein the second VMI version of the CT data is a VMI at an energy below 40 keV.

**11.** The system of claim 1, wherein the at least one hardware processor is further configured to:
receive the CT data from one of a CT scanner or memory.

**12.** The system of claim 11, wherein the CT scanner is one of a dual-energy computed tomography scanner that is configured to generate the CT data or a photon counting detector computed tomography (PCD-CT) scanner that is configured to generate the CT data.

**13.** The system of claim 11, wherein the system further comprises the CT scanner.

**14.** The system of claim 11, wherein the at least one hardware processor is further configured to:
receive the CT data from the CT scanner over a wide area network.

**Patentansprüche**

**1.** System zur Umwandlung von Computertomographiedaten, wobei das System umfasst:

mindestens einen Hardwareprozessor, der konfiguriert ist, um
eine Eingabe zu empfangen, die ein bestimmtes Röntgenenergieniveau angibt, bei dem ein virtuelles monoenergetisches Bild (VMI) eines Objekts erzeugt werden soll, das eine Vielzahl von Zielmaterialien mit verbessertem Kontrast aufweist, wobei die Eingabe, die das bestimmte Röntgenenergieniveau angibt, bei dem das VMI des Objekts erzeugt werden soll, eine Eingabe umfasst, die die Vielzahl von Zielmaterialien explizit angibt;
Empfangen von Computertomographiedaten (CT-Daten) eines Objekts;
Identifizieren der bestimmten Röntgenenergie als eine Röntgenenergie, bei der der Kontrast zwischen der Vielzahl von Zielmaterialien maximal ist;
Erzeugen einer VMI-Version der CT-Daten bei der bestimmten Röntgenenergie durch:

Bereitstellen der CT-Daten an ein trainiertes Modell, wobei das trainierte Modell ein trainiertes Convolutional Neural Network (CNN) ist, das unter Verwendung von CT-Daten von einem oder mehreren Objekten, die jeweils mindestens ein Material aus einer Vielzahl von Materialien darstellen, und unter Verwendung von synthetisierten monoenergetischen CT-Bildern von mindestens einem der einen oder mehreren Objekte auf der Grundlage der Röntgenstrahlungsabschwächung der Vielzahl von Materialien trainiert wurde;
Empfangen einer Ausgabe von dem trainierten Modell;
Erzeugen der VMI-Version der CT-Daten auf der Grundlage der Ausgabe des trainierten Modells; und
Veranlassen, dass die VMI-Version der CT-Daten dargestellt wird.

**2.** System nach Anspruch 1, wobei die CT-Daten Multi-Energie-Computertomographiedaten (MECT-Daten) des Objekts umfassen.

**3.** System nach Anspruch 1, wobei die CT-Daten MECT-Projektionsdomänendaten des Objekts umfassen.

**4.** System nach Anspruch 1, wobei das Objekt ein Phantom aufweist, das eine Vielzahl von Proben umfasst, wobei jede der Vielzahl von Proben mindestens ein Material einer Vielzahl von Materialien darstellt.

**5.** Das System nach Anspruch 1, wobei das trainierte Modell ein Modell der kleinsten Quadrate ist.

**6.** Das System nach Anspruch 5, wobei die CT-Daten MECT-Bilddaten sind.

**7.** Das System nach Anspruch 1, wobei die Vielzahl von Zielmaterialien mindestens zwei der folgenden umfasst:

graue Substanz;
weiße Substanz;
ein Jodkontrastmittel;
Kalzium;
eine Mischung aus Blut und Jod;
Fettgewebe;
Hydroxylapatit;
Lunge;
Leber;
Muskel; oder
Blut.

**8.** System nach Anspruch 1, wobei der mindestens eine Hardwareprozessor ferner konfiguriert ist, um eine zweite VMI-Version der CT-Daten bei einer zweiten bestimmten Röntgenenergie zu erzeugen.

**9.** System nach Anspruch 8, wobei die zweite VMI-Version der CT-Daten eine 50-keV-VMI ist.

**10.** System nach Anspruch 8, wobei die zweite VMI-Version der CT-Daten eine VMI mit einer Energie unter 40 keV ist.

**11.** System nach Anspruch 1, wobei der mindestens eine Hardwareprozessor ferner konfiguriert ist, um:
die CT-Daten von einem CT-Scanner oder einem Speicher zu empfangen.

**12.** System nach Anspruch 11, wobei der CT-Scanner entweder ein Dual-Energie-Computertomographiedatenscanner, der zum Erzeugen der CT-Daten konfiguriert ist, oder ein Photonen-Zähl-Detektor-Computertomographiedatenscanner (PCD-CT-Scanner) ist, der zum Erzeugen der CT-Daten konfiguriert ist.

**13.** Das System nach Anspruch 11, wobei das System ferner den CT-Scanner umfasst.

**14.** Das System nach Anspruch 11, wobei der mindestens eine Hardwareprozessor ferner konfiguriert ist, um:
die CT-Daten von dem CT-Scanner über ein Wide-Area-Netzwerk zu empfangen.

**Revendications**

**1.** Système de transformation de données de tomodensitométrie, le système comprenant :
au moins un processeur matériel configuré pour :

recevoir une entrée indicative d'un niveau d'énergie de rayons X particulier à laquelle générer une image monoénergétique virtuelle (VMI) d'un sujet qui inclut une pluralité de matériaux cibles avec un contraste amélioré, dans lequel l'entrée indicative du niveau d'énergie de rayons X particulier à laquelle générer la VMI du sujet comprend une entrée indiquant explicitement la pluralité de matériaux cibles ;
recevoir des données de tomodensitométrie (CT) d'un sujet ;
identifier l'énergie de rayons X particulière comme une énergie de rayons X à laquelle le contraste entre la pluralité de matériaux cibles est maximisé ;
générer une version VMI des données CT à l'énergie de rayons X particulière par :

la fourniture des données CT à un modèle entraîné, dans lequel le modèle entraîné est un réseau neuronal convolutionnel entraîné (CNN) qui a été entraîné à l'aide de données CT d'un ou de plusieurs sujets, chacun représentant au moins un matériau d'une pluralité de matériaux, et à l'aide d'images CT monoénergétiques synthétisées d'au moins l'un des un ou plusieurs sujets sur la base d'une atténuation par rayons X de la pluralité de matériaux ;
la réception d'une sortie du modèle entraîné ;
la génération de la version VMI des données CT sur la base de la sortie du modèle entraîné ; et

l'affichage de la version VMI des données CT.

2. Système selon la revendication 1, dans lequel les données de tomodensitométrie comprennent des données d'image de tomodensitométrie multi-énergie (MECT) du sujet.

3. Système selon la revendication 1, dans lequel les données CT comprennent des données de domaine de projection MECT du sujet.

4. Système selon la revendication 1, dans lequel le sujet comporte un fantôme comprenant une pluralité d'échantillons, chacun de la pluralité d'échantillons représentant au moins un matériau d'une pluralité de matériaux.

5. Système selon la revendication 1, dans lequel le modèle entraîné est un modèle des moindres carrés.

6. Système selon la revendication 5, dans lequel les données CT sont des données d'image MECT.

7. Système selon la revendication 1, dans lequel la pluralité de matériaux cibles comprend au moins deux parmi :

matière grise ;
matière blanche ;
milieu de contraste iodé ;
calcium ;
mélange de sang et d'iode ;
tissu adipeux ;
hydroxyapatite ;
poumon ;
foie ;
muscle ; ou
sang.

8. Système selon la revendication 1, dans lequel l'au moins un processeur matériel est en outre configuré pour : générer une seconde version VMI des données CT à une seconde énergie de rayons X particulière.

9. Système selon la revendication 8, dans lequel la seconde version VMI des données CT est une VMI 50 keV.

10. Système selon la revendication 8, dans lequel la seconde version VMI des données CT est une VMI à une énergie inférieure à 40 keV.

11. Système selon la revendication 1, dans lequel l'au moins un processeur matériel est en outre configuré pour : recevoir les données CT à partir d'un scanner CT ou d'une mémoire.

12. Système selon la revendication 11, dans lequel le scanner CT est l'un parmi un scanner de tomodensitométrie à double énergie qui est configuré pour générer les données CT ou un scanner de tomodensitométrie à détecteur de comptage de photons (PCD-CT) qui est configuré pour générer les données CT.

13. Système selon la revendication 11, dans lequel le système comprend en outre le scanner CT.

14. Système selon la revendication 11, dans lequel l'au moins un processeur matériel est en outre configuré pour : recevoir les données CT du scanner CT sur un réseau étendu.

FIG. 1

FIG. 2

EP 4 322 856 B1

EP 4 322 856 B1

301

300

306
Material mass densities of phantom(s)

308
Density patches

302
MECT images of phantom(s)

304
MECT Patches

310

Untrained CNN

I+Blood
I+Blood
HA
Adipose
HA
I+Blood
I+Blood
Blood
HA
HA

322
Unlabeled MECT image

324

Trained CNN

326
Predicted densities

312
Predicted densities

314
Loss calculation

328
Image processing (e.g., a VMI(s))

330
Transformed Image(s)

- - - training - - - -
——— trained ———

FIG. 3

400

RECEIVE MULTI-ENERGY COMPUTED TOMOGRAPHY (MECT) IMAGES OF
AN OBJECT WITH KNOWN DENSITIES (E.G., A CT PHANTOM)
402

GENERATE MATERIAL MASS DENSITY MASKS FOR EACH MECT IMAGE
BASED ON KNOWN MASS DENSITIES
404

EXTRACT PATCHES OF EACH MECT IMAGE FOR USE AS TRAINING DATA
OR TEST DATA
406

ADD NOISE TO PATCHES TO SIMULATE LOWER DOSE SCAN
408

PAIR PATCHES WITH CORRESPONDING PORTION OF MATERIAL MASS
DENSITY MASK
410

TRAIN CONVOLUTIONAL NEURAL NETWORK (CNN) TO PREDICT
MATERIAL MASS DENSITIES USING PATCHES AND CORRESPONDING
PORTION OF MATERIAL MASS DENSITY MASK
412

RECEIVE UNLABELED MECT IMAGE OF A SUBJECT (E.G., A PATIENT)
414

PROVIDE THE UNLABELED MECT SCAN TO THE TRAINED CNN
416

RECEIVE, FROM THE CNN, A PREDICTED MATERIAL MASS DENSITY
DISTRIBUTION FOR EACH PIXEL OF THE UNLABELED MECT SCAN
418

GENERATE A VIRTUAL IMAGE(S) USING THE PREDICTED MATERIAL MASS
DENSITY DISTRIBUTION
420

PRESENT THE VIRTUAL IMAGE(S) AND/OR THE UNLABELED MECT IMAGE
422

FIG. 4

FIG. 5

FIG. 6

700

RECEIVE MULTI-ENERGY COMPUTED TOMOGRAPHY (MECT) IMAGES OF AN OBJECT(S) WITH KNOWN DENSITIES (E.G., A CT PHANTOM) — 702

GENERATE SYNTHETIC MONOENERGETIC CT VALUES FOR EACH MECT SCAN AND EACH VMI ENERGY — 704

PAIR PATCHES WITH CORRESPONDING PORTION OF SIMULATED MONOENERGETIC CT VALUES — 706

TRAIN A FIRST CONVOLUTIONAL NEURAL NETWORK (CNN) TO GENERATE ONE OR MORE VIRTUAL MONOCHROMATIC IMAGES (VMI) USING THE PATCHES AND CORRESPONDING SIMULATED MONOCHROMATIC CT VALUES — 708

RECEIVE MECT IMAGES OF MULTIPLE OBJECTS — 710

PAIR PATCHES WITH ROUTINE-DOSE CT — 712

TRAIN A SECOND CNN TO GENERATE A REFINED VMI USING THE VMI FROM THE FIRST CNN AND CORRESPONDING COMPOSITE CT DATA — 714

RECEIVE UNLABELED MECT SCAN OF A SUBJECT (E.G., A PATIENT) — 716

PROVIDE THE UNLABELED MECT SCAN TO THE FIRST TRAINED CNN — 718

RECEIVE, FROM THE SECOND CNN, A VMI(S) BASED ON THE MECT SCAN — 720

PRESENT THE VIRTUAL IMAGE(S) — 722

FIG. 7

FIG. 8

FIG. 9

FIG. 10

EP 4 322 856 B1

FIG. 11

FIG. 12

Clinical Mix Image
W/L: 80/40 HU

40 keV VMI
W/L: 80/50 HU

18 keV VMI
W/L: 80/70 HU

CNN

FIG. 13

1400

DETERMINE CT NUMBER (E.G., IN HOUNSFIELD UNITS) OF VARIOUS MATERIALS OF INTEREST AT VARIOUS ENERGY LEVELS — 1402

DETERMINE, FOR ONE OR MORE CLINICAL TASKS, AN ENERGY LEVEL THAT PRODUCES IMPROVED CONTRAST — 1404

GENERATE A MODEL THAT PRODUCES A VMI(S) FROM MECT DATA (E.G., USING PAIRED PATCHES AND SIMULATED MONOENERGETIC CT VALUES) — 1406

RECEIVE CT DATA (E.G., MECT DATA, ONE OR MORE VIRTUAL MONOENERGETIC IMAGES) OF SUBJECT — 1408

RECEIVE INPUT INDICATIVE OF AN ENERGY LEVEL(S) AT WHICH TO GENERATE A VMI(S) — 1410

USE THE MODEL(S) TO GENERATE A VMI(S) BASED ON THE RECEIVED CT DATA — 1412

PRESENT THE VIRTUAL MONOENERGETIC IMAGE(S) — 1414

FIG. 14

FIG. 15A

EP 4 322 856 B1

**Gray / White matter contrast**

FIG. 15B

FIG. 16A

FIG. 16B

FIG. 17

Routine radiation dose level

50% of routine radiation dose level

FIG. 18

**Phantom measurement – CT number accuracy at 18 keV**

FIG. 19

FIG. 20A

## CNR between ROIs #1 & #2 in patient images

FIG. 20B

## Profiles of L1 in patient images

FIG. 20C

FIG. 21A

## CNR between ROIs #3 & #4 in patient images

FIG. 21B

## Profiles of L2 in patient images

FIG. 21C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63221427

**Non-patent literature cited in the description**

- Deep-learning-based direct synthesis of low-energy virtual monoenergetic images with multi-energy CT. *GONG HAO ET AL, JOURNAL OF MEDICAL IMAGING, SOCIETY OF PHOTO-OPTICAL IN-STRUMENTATION ENGINEERS*, vol. 8 (5)

- **KINGMA et al.** *Adam: A Method for Stochastic Optimization*, 2014